# EUROPEAN PATENT APPLICATION

(11) **EP 4 400 036 A1**
(43) Date of publication of application: **17.07.2024**
(21) Application number: 22866528.7
(22) Date of filing: 02.09.2022
(51) Int. Cl.: A61B 5/0205

(54) **WEARABLE MOBILE MONITORING DEVICE, MONITORING SYSTEM, AND DATA TRANSMISSION METHOD**

(30) Priority: 08.09.2021 CN 202111051456
(71) Applicant: Shenzhen Mindray Bio-Medical Electronics Co., Ltd, Shenzhen, Guangdong 518057 (CN)
(72) Inventor: NIE, Pengpeng, Shenzhen, Guangdong 518057 (CN); CHEN, Weikui, Shenzhen, Guangdong 518057 (CN); CHEN, Changgen, Shenzhen, Guangdong 518057 (CN); XIA, Hengxing, Shenzhen, Guangdong 518057 (CN); ZHANG, Kui, Shenzhen, Guangdong 518057 (CN); ZHANG, Zhenqi, Shenzhen, Guangdong 518057 (CN); XU, Li, Shenzhen, Guangdong 518057 (CN)
(74) Representative: KIPA AB
(86) International application number: PCT/CN2022/116917
(87) International publication number: WO 2023/036065

(57) **Abstract**

A wearable mobile monitoring device, a monitoring system, and a data transmission method. The device comprises: a sensor, configured to collect a signal representing at least one vital sign parameter of a human body; and a processor, configured to: obtain first monitoring data on the basis of the signal collected by the sensor; receive second monitoring data from a mobile monitoring unit by means of a first communication connection established between a first wireless communication unit and the mobile monitoring unit worn on the human body; under a first preset condition, transmit the first monitoring data and the second monitoring data to a near-end device by means of a second communication connection established between the first wireless communication unit and the near-end device outside the human body, the second communication connection and the first communication connection being based on a same wireless communication technology; and under a second preset condition, transmit the first monitoring data and the second monitoring data to a remote device by means of a third communication connection established between a second wireless communication unit and the remote device, the third communication connection and the first communication connection being based on different wireless communication technologies.

## Description

### TECHNICAL FIELD

The disclosure relates to a technical field of medical monitoring device, and more particularly to a wearable mobile monitoring device, a monitoring system, and a data transmission method.

### BACKGROUND

In wearable medical devices, due to size constraints, it is difficult for batteries to have a large capacity, so that the battery capacity is small. On one hand, users usually expect wearable medical devices to be used for as long as possible, and therefore have higher requirements for the battery life of wearable medical devices. On the other hand, wearable medical devices usually need to interact with other devices to feedback, record, and store monitoring data, while wireless transmission is the main data transmission method. However, wireless transmission requires more power consumption. Therefore, under the influence of comprehensive factors, such as low battery capacity, high endurance requirement, and high-power consumption of wireless data transmission, how to implement appropriate wireless networking technology for wearable medical devices and their interactive devices, so as to improve the endurance of wearable medical devices, has become a problem that needs to be solved.

### SUMMARY

This disclosure provides a wearable mobile monitoring device, a monitoring system and a data transmission method, aiming at the above problems.

According to one aspect of this disclosure, a wearable mobile monitoring device is provided, including a sensor, a processor, a first wireless communication unit and a second wireless communication unit; wherein the sensor is configured to acquire a signal which characterizes at least one vital sign parameter of a human body, the processor is configured to perform following operation: obtaining first monitoring data based on the signal which is acquired by the sensor; controlling the mobile monitoring device to receive, from a mobile monitoring unit which is worn on the human body, second monitoring data, through first communicative connection, which is established between the first wireless communication unit and the mobile monitoring unit; transmitting, under a first preset condition to a near-end device which is apart from the human body, the first monitoring data and the second monitoring data, through second communicative connection, which is established between the first wireless communication unit and the near-end device, wherein the second communicative connection and the first communicative connection are based on a same wireless communication technology, the near-end device is located within a preset range; transmitting, under a second preset condition to a far-end device, the first monitoring data and the second monitoring data, through third communicative connection, which is established between the second wireless communication unit and the far-end device, wherein the third communicative connection and the first communicative connection are based on different wireless communication technologies, and the far-end device is located outside the preset range.

According to another aspect of this disclosure, a monitoring system is provided, including a wearable mobile monitoring device and a mobile monitoring unit which are worn on a human body, and a near-end device and a far-end device which are apart from the human body; wherein the mobile monitoring device is configured to monitor first monitoring data of the human body, the mobile monitoring unit is configured to monitor second monitoring data of the human body, the near-end device and the far-end device are configured to obtain the first monitoring data and the second monitoring data; the mobile monitoring device is further configured to perform following operations: receiving, from the mobile monitoring unit, the second monitoring data, through first communicative connection which is established with the mobile monitoring unit; transmitting, under a first preset condition to the near-end device, the first monitoring data and the second monitoring data, through second communicative connection, which is established with the near-end device, wherein the second communicative connection and the first communicative connection are based on a same wireless communication technology, the near-end device is located within a preset range; transmitting, under a second preset condition to the far-end device, the first monitoring data and the second monitoring data, through third communicative connection, which is established with the far-end device, wherein the third communicative connection and the first communicative connection are based on different wireless communication technologies, and the far-end device is located outside the preset range.

According to yet another aspect of this disclosure, a data transmission method which is applicable to a wearable mobile monitoring device; wherein the data transmission method includes: acquiring a signal which characterizes at least one vital sign parameter of a human body; obtaining first monitoring data based on the signal; receiving, from a mobile monitoring unit which is worn on the human body, second monitoring data, through first communicative connection, which is established with the mobile monitoring unit; transmitting, under a first preset condition to a near-end device which is apart from the human body, the first monitoring data and the second monitoring data, through second communicative connection, which is established with the near-end device, wherein the second communicative connection and the first communicative connection are based on a same wireless communication technology, the near-end device is located within a preset range; transmitting, under a second preset condition to a far-end device, the first monitoring data and the second monitoring data, through third communicative connection, which is established with the far-end device, wherein the third communicative connection and the first communicative connection are based on different wireless communication technologies, and the far-end device is located outside the preset range.

In the mobile monitoring device, monitoring system and data transmission method of this disclosure, the mobile monitoring device can communicate with another mobile monitoring unit worn on the human body and a near-end device apart from the human body based on the same wireless communication technology, thus reducing a complexity of wireless networking technology, saving power consumption, improving battery life, and also providing further possibilities for device miniaturization. In addition, the mobile monitoring device, monitoring system and data transmission method of this disclosure can also communicate with a far-end device based on another communication technology, so as to satisfy data transmission requirements in various scenarios.

### BRIEF DESCRIPTION OF THE DRAWINGS

FIG. 1 shows a structural block diagram of a wearable mobile monitoring device according to an embodiment of this disclosure.
FIG. 2 shows a structural block diagram of a monitoring system according to an embodiment of this disclosure.
FIG. 3 shows an exemplary diagram of a wireless topology of a monitoring system according to an embodiment of this disclosure.
FIG. 4 shows a data transmission diagram of a monitoring system according to an embodiment of this disclosure.
FIG. 5 shows a data transmission diagram of a modified example of a monitoring system according to an embodiment of this disclosure.
FIG. 6 shows a schematic flow chart of a data transmission method according to an embodiment of this disclosure.

### DESCRIPTION OF THE PREFERRED EMBODIMENTS

In order to make the purpose, technical solution, and advantages of this disclosure more obvious, the following refers to the attached drawings to describe in detail the exemplary embodiments according to this disclosure. Obviously, the described embodiments are only some of the embodiments of this disclosure, not all of them. It should be understood that this disclosure is not limited by the embodiments described here. Based on the embodiments described in this disclosure, all other embodiments acquired by those skilled in the art without creative work, fall within the protection scope of this disclosure.

In the following description, a large number of specific details are provided to provide a more thorough understanding of this disclosure. However, it is evident to those skilled in the art that this disclosure can be implemented without the need for one or more of these details. In other examples, in order to avoid confusion with this disclosure, some well-known technical characteristics in this field are not described.

It should be understood that this disclosure can be implemented in different forms and should not be limited to the embodiments proposed here. On the contrary, providing these embodiments make the disclosure thorough and complete, and fully convey the scope of this disclosure to those skilled in the art.

The purpose of the terminology used here is only to describe specific embodiments and is not a limitation of this disclosure. When used here, the singular forms of "an", "a", and "the/said" are also intended to include the plural form, unless the context clearly indicates another way. It should also be understood that the terms, such as "including", "and/or", "comprising", when used in this disclosure, determine the existence of the characteristics, integers, steps, operations, components, and/or elements, but do not exclude the existence or addition of one or more other characteristics, integers, steps, operations, components, and/or elements. When used here, the term "and/or" includes any and all combinations of related listed items.

In order to fully understand this disclosure, detailed steps and detailed structures are provided in the following description to explain the technical solution proposed in this disclosure. The optional embodiments of this disclosure are described in detail below, however, in addition to these detailed description, this disclosure may also have other embodiments.

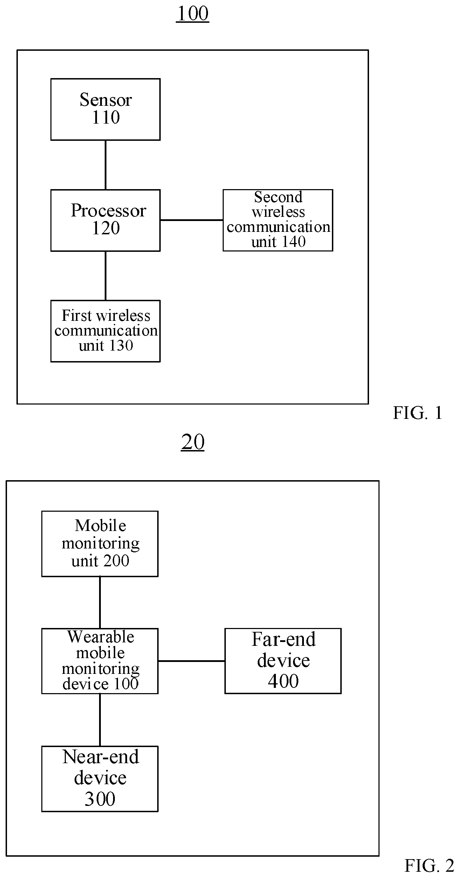

FIG. 1 shows a structural block diagram of a wearable mobile monitoring device 100 according to an embodiment of this disclosure. As shown in FIG. 1, the mobile monitoring device 100 includes a sensor 110, a processor 120, a first wireless communication unit 130 and a second wireless communication unit 140. The sensor 110 is configured to acquire a signal which characterizes at least one vital sign parameter of a human body. The processor 120 is configured to perform following operations: obtaining first monitoring data based on the signal which is acquired by the sensor 110; controlling the mobile monitoring device 100 to receive, from a mobile monitoring unit which is worn on the human body, second monitoring data, through first communicative connection, which is established between the first wireless communication unit 130 and the mobile monitoring unit; transmitting, under a first preset condition to a near-end device which is apart from the human body, the first monitoring data and the second monitoring data, through second communicative connection, which is established between the first wireless communication unit 130 and the near-end device; wherein the second communicative connection and the first communicative connection are based on a same wireless communication technology, the near-end device is located within a preset range; transmitting, under a second preset condition to a far-end device, the first monitoring data and the second monitoring data, through third communicative connection, which is established between the second wireless communication unit 140 and the far-end device; wherein the third communicative connection and the first communicative connection are based on different wireless communication technologies, and the far-end device is located outside the preset range.

In an embodiment of this disclosure, the mobile monitoring device 100 includes the first wireless communication unit 130 and the second wireless communication unit 140, wherein the first wireless communication unit 130 supports a first wireless communication technology, and the second wireless communication unit 140 supports a second wireless communication technology. The first wireless communication technology and the second wireless communication technology are different wireless communication technologies. Therefore, the first communicative connection with the mobile monitoring unit worn on the human, which connection is established by the mobile monitoring device 100 through the first wireless communication unit 130, and the second communicative connection with the near-end device apart from the human body, which connection is established by the mobile monitoring device 100 through the first wireless communication unit 130, are based on the same wireless communication technology. While the third communicative connection with the far-end device, which connection is established by the mobile monitoring device 100 through the second wireless communication unit 140 is based on a different wireless communication technology, when compared with the first communicative connection and the second communicative connection. Since the mobile monitoring device 100 is a wearable device, it is usually worn on the human body to work. The mobile monitoring device 100 is close to the mobile monitoring unit worn on the human body (i.e., another wearable mobile monitoring device 100, which is named as a mobile monitoring unit for distinguishing). The near-end device is located within a preset range, that is, the near-end device is a device which is relatively close to the mobile monitoring device 100. The far-end device is located outside the preset range, that is, the far-end device is a device which is relatively far from the mobile monitoring device 100. Meanwhile, the mobile monitoring device 100 respectively communicates with the mobile monitoring unit worn on the human body and the near-end device which are apart from the human body through the first wireless communication unit 130, and communicates with the far-end device through the second wireless communication unit 140. Therefore, it is easy to understand that the first wireless communication technology supported by the first wireless communication unit 130 is a technology related to near-field communication, and the second wireless communication technology supported by the second wireless communication unit 140 is a technology related to far-field communication. Therefore, the communication between the mobile monitoring device 100 and the mobile monitoring unit worn on the human body, as well as the communication between the mobile monitoring device 100 and the near-end device which are apart from the human body under the first preset condition, are both through a wireless communication technology related to near-field communication. Only the communication between the mobile monitoring device 100 and the far-end device under the second preset condition, is through a wireless communication technology related to far-field communication. Compared with the far-field wireless communication technology, the near-field wireless communication technology consumes less power, because the communication distance is shorter, so the required transmission power is smaller. Therefore, compared with a wearable mobile monitoring device that uses a far-field wireless communication technology to communicate with both near-end and far-end devices, the mobile monitoring device 100 according to the embodiment of this disclosure can greatly reduce power consumption, thereby improving battery life, and providing further possibilities for device miniaturization. In addition, as the mobile monitoring device 100 according to the embodiment of this disclosure communicates with the mobile monitoring unit worn on the human body and the near-end device apart from the human body based on the same wireless communication technology, such that the mobile monitoring device 100, the mobile monitoring unit worn on the human body and the near-end device apart from the human body can complete their wireless network deployments within a same body area network, thus simplifying the complexity of network deployment. Moreover, since the mobile monitoring device 100 according to the embodiment of this disclosure also has a second wireless communication unit 140 that supports the far-field wireless communication technology, the mobile monitoring device 100 can also communicate with the far-end device, thereby satisfying data transmission requirements in different scenarios.

In an embodiment of this disclosure, the aforementioned first preset condition may include: a communication quality for current communication between the mobile monitoring device 100 and the near-end device through the first wireless communication unit 130, satisfies a preset standard. In this embodiment, when the communication quality for current communication between the mobile monitoring device 100 and the near-end device through the first wireless communication unit 130, satisfies the preset standard, the mobile monitoring device 100 communicates with the near-end device through the first wireless communication unit 130, so as to transmit, to the near-end device, the first monitoring data which is monitored by itself and the second monitoring data which is monitored by the mobile monitoring unit. Wherein, "satisfying the preset standard" can be understood as the mobile monitoring device 100 is capable of communicating with the near-end device through the first wireless communication unit 130, or the communication quality between the mobile monitoring device 100 and the near-end device is reliable. For example, it can be guaranteed that there is no packet loss in data transmitted from the mobile monitoring device 100 to the near-end device through the first wireless communication unit 130, and each data transmission can receive a response which indicates that the data is successfully received, etc. Whenever the first preset condition is satisfied, the mobile monitoring device 100 can communicate with the near-end device through the first wireless communication unit 130, so as to reduce power consumption and improve battery life, as much as possible.

In an embodiment of this disclosure, the aforementioned second preset condition may include: a communication quality for current communication between the mobile monitoring device 100 and the near-end device through the first wireless communication unit 130, does not satisfy a preset standard. In this embodiment, when the communication quality of current communication between the mobile monitoring device 100 and the near-end device through the first wireless communication unit 130, does not satisfy the preset standard, the mobile monitoring device 100 communicates with the far-end device through the second wireless communication unit 140, so as to transmit, to the far-end device, the first monitoring data which is monitored by itself and the second monitoring data which is monitored by the mobile monitoring unit. Wherein, "does not satisfy the preset standard" can be understood as the mobile monitoring device 100 is not capable of communicating with the near-end device through the first wireless communication unit 130, or the communication quality between the mobile monitoring device 100 and the near-end device is not reliable. For example, there is a packet loss in data transmitted from the mobile monitoring device 100 to the near-end device through the first wireless communication unit 130, or no response is received based on the data transmission, etc. Whenever the second preset condition is satisfied, the mobile monitoring device 100 can communicate with the far-end device through the second wireless communication unit 140, so as to guarantee outgoing transmission of monitoring data.

In an embodiment of this disclosure, when transmitting the first monitoring data and the second monitoring data, the processor 120 may also be configured to transmit, under a third preset condition, the first monitoring data and the second monitoring data, through the second communicative connection or the third communicative connection in a continuous transmission mode; or transmit under a preset condition which is other than the third present condition, the first monitoring data and the second monitoring data, through the second communicative connection or the third communicative connection in a continuous transmission mode or a discontinuous transmission mode; wherein the continuous transmission mode refers to transmitting data in multiple continuous transmission cycles, and the discontinuous transmission mode refers to transmitting data only in a portion of transmission cycle(s) of multiple continuous transmission cycles. In this embodiment, when the mobile monitoring device 100 transmits data to the near-end device or the far-end device, it provides two data transmission modes, that is, the continuous transmission mode and the discontinuous transmission mode. The continuous transmission mode, as the name implies, is to transmit data continuously and uninterruptedly. For example, a transmission cycle (such as 1 second, etc., wherein the transmission cycle is a time period, which is required to transmit data once), is preset, then in each transmission cycle, the continuous transmission mode transmits data. The discontinuous transmission mode, as the name implies, is to transmit data discontinuously and intermittently. That is, for the preset transmission cycle, data is not transmitted in every transmission cycle, but is transmitted in some transmission cycle(s), and is not transmitted in other transmission cycle(s). Based on these two data transmission modes, under the third preset condition, the continuous transmission mode is used to transmit to the near-end device or the far-end device (transmitting to the near-end device or the far-end device depends on the first preset condition or second preset condition mentioned above), the first monitoring data, which is monitored by the mobile monitoring device 100 itself, and the second monitoring data, which is monitored by the mobile monitoring unit (worn by the human body of the patient) and then acquired by the mobile monitoring device 100; while under the preset condition which is other than the third present condition, the discontinuous transmission mode is used to transmit to the near-end device or the far-end device (transmitting to the near-end device or the far-end device depends on the first preset condition or second preset condition mentioned above), the first monitoring data, which is monitored by the mobile monitoring device 100 itself, and the second monitoring data, which is monitored by the mobile monitoring unit (worn by the human body of the patient) and then acquired by the mobile monitoring device 100. In such a way, power consumption can be saved.

In an embodiment of this disclosure, the aforementioned third preset condition may also include at least one of a condition which reflects a human health risk, a condition which reflects a parameter transmission mode, and a condition which reflects a user instruction. In this embodiment, the data transmission mode can be determined by combining at least one of the condition which reflects a human health risk, the condition which reflects a parameter transmission mode, and the condition which reflects a user instruction, so as to satisfy different application scenarios and/or user requirements. These three conditions are described below.

For the condition which reflects a human health risk, it can be understood that occurrence of such condition reflects that human health has a risk. Accordingly, when no condition which reflects a human health risk, exists, the discontinuous transmission mode is used to transmit data, so that the data transmission mode can be determined according to health and physiological requirements of the patient, which can save power consumption as much as possible while avoiding the occurrence of patient risk event and ensuring the life safety of patient. Of course, when no condition which reflects a human health risk, exists, the continuous transmission mode can also be used to transmit data, which can satisfy an application scenario, where the user does not care about battery life and pays more attention to monitoring.

In an embodiment of this disclosure, the aforementioned condition which reflects a human health risk may further include a monitoring time of the mobile monitoring device 100 for the human body, does not reach a preset time threshold; and/or, the first monitoring data and/or the second monitoring data indicate(s) that the human body is currently in a risk state. In this embodiment, a health risk situation of the human body is determined based on the monitoring time and/or the monitoring data. For example, for a patient who has undergone surgery, a period from when the surgery completes to a certain time period after the surgery (such as within 8 hours after surgery) is usually a high-risk period. The condition which reflects a human health risk, exists during this period, that is, during this period, the first monitoring data and the second monitoring data are transmitted in the continuous transmission mode, so as to ensure the life safety of patient. On the contrary, when the monitoring time for the human body reaches the preset time threshold (such as 8 hours have passed after the surgery), then the patient has usually passed the high-risk period and can be regarded as that the condition which reflects a human health risk, does not exist. That is, at this time, the first monitoring data and the second monitoring data are transmitted in the discontinuous transmission mode, so as to save power consumption. For another example, after the mobile monitoring device 100 acquires the first monitoring data and the second monitoring data, it can perform a simple analysis on the monitoring data, for example, determining whether the monitoring data is within a safety threshold range. If the monitoring data exceeds the safety threshold range, it indicates that the human body is currently in a risk state, and it is considered that the condition which reflects a human health risk exists at this time, that is, the continuous transmission mode is used to transmit the first monitoring data and the second monitoring data during this period to ensure the life safety of patient. Conversely, if the monitoring data is determined as within the safety threshold range, it indicates that the human body is not currently in a risk state, which means the condition which reflects a human health risk does not exist at this time, that is, the discontinuous transmission mode is used to transmit the first monitoring data and the second monitoring data during this period, so as to save power consumption.

For a condition which reflects a parameter transmission mode, it is easy to understand that occurrence of this condition reflects a specific parameter transmission mode, so that a corresponding data transmission mode can be determined based on the parameter transmission mode reflected in this condition. For example, the aforementioned condition which reflects a parameter transmission mode may include the first monitoring data and the second monitoring data are transmitted through the first wireless communication unit 130. In this embodiment, since the monitoring data is transmitted through the first wireless communication unit 130, and as described above, the first wireless communication technology supported by the first wireless communication unit 130 is a technology related to near-field communication. As the power consumption of the technology related to near-field communication is low, so in this case, the continuous transmission mode can be used to transmit, to the near-end device, the first monitoring data, which is monitored by the mobile monitoring device 100 itself, and the second monitoring data, which is monitored by the mobile monitoring unit (worn by the human body of the patient) and then acquired by the mobile monitoring device 100. That is, when the mobile monitoring device 100 transmits, through the first wireless communication unit, the monitoring data to the near-end device, even if the continuous transmission mode is adopted, more complete monitoring data records can be ensured at the near-end device without consuming too much power, which is conducive to real-time viewing, monitoring or reviewing of the patient condition at the near-end device. On the contrary, if the first monitoring data and the second monitoring data are transmitted not through the first wireless communication unit 130, but through the second wireless communication unit 140, this can be considered as a condition which is other than the third preset condition, and as described above, the second wireless communication technology supported by the second wireless communication unit 140 is a technology related to far-field communication. Since the power consumption of the technology related to far-field communication is high, in this case, the discontinuous transmission mode can be used to transmit, to the far-end device, the first monitoring data, which is monitored by the mobile monitoring device 100 itself, and the second monitoring data, which is monitored by the mobile monitoring unit (worn by the human body of the patient) and then acquired by the mobile monitoring device 100, so as to save power consumption.

For the condition which reflects a user instruction, it is also easy to understand that the condition reflects a user requirement, so that the corresponding data transmission mode can be determined according to the user instruction in this condition. For example, in an embodiment of this disclosure, the aforementioned condition which reflects a user instruction may include a user instruction, which instructs to transmit the first monitoring data and the second monitoring data in the continuous transmission mode, is received by the mobile monitoring device 100. In this embodiment, if a user instruction, which instructs to transmit the first monitoring data and the second monitoring data in the continuous transmission mode, is received, the mobile monitoring device 100 transmits, to the near-end device or the far-end device in the continuous transmission mode based on the user instruction, the first monitoring data, which is monitored by the mobile monitoring device 100 itself, and the second monitoring data, which is monitored by the mobile monitoring unit (worn by the human body of the patient) and then acquired by the mobile monitoring device 100, so as to satisfy the user requirement. This embodiment is generally applied to an application scenario where the user does not care about battery life but is more concerned about monitoring.

Furthermore, when at least two conditions are combined to determine the data transmission mode, a priority of each condition may be preset. For example, among the above three conditions, the condition which reflects a human health risk has a highest priority. In this way, when the condition which reflects a human health risk, exists, the continuous transmission mode is used to transmit data regardless of other conditions; when the condition which reflects a human health risk, does not exist, the transmission mode of the monitoring data can be determined in combination with the condition which reflects a parameter transmission mode and/or the condition which reflects a user instruction. In this way, different communication scenarios and/or different user requirements can be satisfied, while ensuring life safety of patient.

In an embodiment of this disclosure, the mobile monitoring device 100 may also include a memory (not shown in FIG. 1), and the processor 120 may also be configured to store, in the memory, the first monitoring data and the second monitoring data, when the second communicative connection between the mobile monitoring device 100 and the near-end device is disconnected. In this embodiment, the mobile monitoring device 100 itself may have a storage function for the monitoring data. When the mobile monitoring device 100 is unable to transmit to the near-end device, the first monitoring data which is acquired through its own monitoring and the second monitoring data which is acquired from another mobile monitoring unit worn on the human body, the mobile monitoring device 100 can use its own memory to store the first monitoring data and the second monitoring data, so as to ensure the complete preservation of the monitoring data. Further, the processor 120 may also be configured to transmit, to the near-end device, the first monitoring data and the second monitoring data which are stored in the memory, through the second communicative connection between the mobile monitoring device 100 and the near-end device, when the second communicative connection is restored. In this embodiment, when the second communicative connection between the mobile monitoring device 100 and the near-end device is restored, the mobile monitoring device 100 may transmit, to the near-end device, the first monitoring data and the second monitoring data, which are stored during a disconnection of the second communicative connection, so as to guarantee the integrity of monitoring data at the near-end device.

In an embodiment of this disclosure, the mobile monitoring device 100 may also include a display screen (not shown in FIG. 1), which may be configured to display the first monitoring data and/or the second monitoring data. In this embodiment, the mobile monitoring device 100 itself can display the first monitoring data which is acquired through its own monitoring and the second monitoring data which is acquired from another mobile monitoring unit worn on the human body, so as to facilitate the patient itself to view.

In an embodiment of this disclosure, the first wireless communication unit 130 mentioned above may include a Bluetooth Low Energy (BLE for short) communication module or a Medical Body Area Network (MBAN for short) communication module. Correspondingly, the first wireless communication technology supported by the first wireless communication unit 130 may include a BLE wireless communication technology or an MBAN wireless communication technology. Correspondingly, the first communicative connection established through the first wireless communication unit 130 with the mobile monitoring unit worn on the human body may include BLE wireless communicative connection or MBAN wireless communicative connection, the second communicative connection established through the first wireless communication unit 130 with the near-end device apart from the human body may include BLE wireless communicative connection or MBAN wireless communicative connection.

In an embodiment of this disclosure, the second wireless communication unit 140 mentioned above may include at least one of a Wireless Fidelity (WIFI for short) communication module, a Wireless Medical Telemetry Services (WMTS for short) communication module, and a cellular network communication module. Correspondingly, the second wireless communication technology supported by the second wireless communication unit 140 may include at least one of a WIFI wireless communication technology, a WMTS wireless communication technology, and a cellular network communication technology. Correspondingly, the third communicative connection established with the far-end device through the second wireless communication unit 140 may include at least one of WIFI wireless communicative connection, WMTS wireless communicative connection, and cellular communicative connection.

In an embodiment of this disclosure, the mobile monitoring device 100 may be a monitoring device that can be worn on the human body and acquire a signal which characterizes at least one vital sign parameter of the human body, such as a monitoring device worn on the wrist to monitor blood oxygen, and so on. The mobile monitoring unit can be another monitoring device worn on the human body to acquire a signal which characterizes at least one vital sign parameter of the human body, such as a monitoring device worn on the neck and chest of the human body for monitoring an ECG signal, a monitoring device worn on upper arms of the human body for monitoring blood pressure, etc. In an embodiment of this disclosure, the aforementioned near-end device may be a medical device (such as a bedside monitor, ultrasound device, etc.) in the same ward as the mobile monitoring device 100, or may be a non-medical electronic device (such as mobile phones, computers, etc.), which is close to the mobile monitoring device 100. In an embodiment of this disclosure, the aforementioned far-end device can be various systems in the hospital where the patient is located, such as a central station at a nurse desk, etc., or other non-medical remote electronic devices, such as a client of telemedicine service, etc.

In an embodiment of this disclosure, when the mobile monitoring device 100 transmits the first monitoring data and the second monitoring data to the far-end device, it may directly or indirectly transmit the data to the far-end device. The indirect transmission to the far-end device may refer to that the mobile monitoring device 100 establishes the aforementioned third communicative connection with a forwarding device, transmits the first monitoring data and the second monitoring data to the forwarding device based on the third communicative connection, and then uses the forwarding device to forward the first monitoring data and the second monitoring data to the far-end device. Wherein, when the second wireless communication unit 140 includes a WIFI communication module and/or a WMTS communication module, the forwarding device may be a WIFI wireless access point (AP for short) device and/or a WMTS AP device. When the second wireless communication unit 140 includes a cellular communication module, the forwarding device may be a base station. In addition, when the second wireless communication unit 140 includes both a WIFI communication module and a WMTS communication module, the WIFI AP device and the WMTS AP device can be two separate entities, or they can be integrated into one device to support both wireless technologies at the same time.

In an embodiment of this disclosure, the mobile monitoring device 100 may further include a proximity-based communication unit (not shown), and the processor 120 is further configured to perform following operations: acquiring, from the mobile monitoring unit, first connection information, in a contactless manner through the proximity-based communication unit, or transmitting, to the mobile monitoring unit, second connection information, in a contactless manner through the proximity-based communication unit; and establishing, based on the first connection information or the second connection information, the first communicative connection with the mobile monitoring unit, through the first wireless communication unit 130; or acquiring, from the near-end device, third connection information, in a contactless manner through the proximity-based communication unit, or transmitting, to the near-end device, fourth connection information, in a contactless manner through the proximity-based communication unit; and establishing, based on the third connection information or the fourth connection information, the second communicative connection with the near-end device, through the first wireless communication unit 130.

In this embodiment, the mobile monitoring device 100 includes at least one proximity-based communication unit. Based on the proximity-based communication unit, the processor 120 of the mobile monitoring device 100 can acquire the first connection information from the mobile monitoring unit in a contactless manner. The first connection information is pairing information between the mobile monitoring device 100 and the mobile monitoring unit. Alternatively, the processor 120 of the mobile monitoring device 100 can transmit the second connection information to the mobile monitoring unit in the contactless manner, and the second connection information is pairing information between the mobile monitoring device 100 and the mobile monitoring unit. In this way, the mobile monitoring device 100 and the mobile monitoring unit have the same pairing information or the pairing information with a corresponding relationship (i.e., the aforementioned first connection information or the second connection information). Based on the pairing information, the first communicative connection mentioned above can be conveniently established between the mobile monitoring unit and the mobile monitoring device 100 through the first wireless communication unit 130, so as to exchange data therebetween. The above pairing process does not require manual binding and pairing of patient information. Instead, the mobile monitoring device 100 and the mobile monitoring unit are paired and connected through the agreed connection information (pairing information) between the mobile monitoring unit and the mobile monitoring device 100. In this way, the user only needs to move the mobile monitoring device 100 and the mobile monitoring unit which serve the same patient close to each other, the pairing desired by the user can be achieved (that is, the user desires pairing between the mobile monitoring unit and the mobile monitoring device 100 currently close to each other rather than pairing between other devices), which greatly simplifies the pairing process between different medical devices which serve the same patient, not only improves the user experience, but also minimizes medical accidents caused by manual operation errors.

In an embodiment of this disclosure, the first connection information includes at least one of: a device identifier (i.e., device ID) or a serial number (SN) of the mobile monitoring unit; one of a communication channel identifier, an IP address, an MAC address, a service set identifier (SSID), Wired Equivalent Privacy (WEP) protocol information, Wi-Fi Protected Access (WPA) protocol information, WPA2 protocol information, WPA3 protocol information, Extensible Authentication Protocol (EAP) information, which are determined by the mobile monitoring unit; and a random pairing code which is generated by the mobile monitoring unit. Wherein, the random pairing code which is generated by the mobile monitoring unit can be one code or a pair of codes. Wherein, the mobile monitoring unit generates one code means that the pairing codes between the mobile monitoring unit and the mobile monitoring device 100 are exactly the same, for example, the code is 1111. The mobile monitoring unit generates a pair of codes means that the mobile monitoring unit and the mobile monitoring device 100 have different pairing codes which have a corresponding relationship. For example, the random pairing codes generated by the mobile monitoring unit are 1111 and 2222 (for example, a pairing relationship list is established through an array), wherein the random pairing code 1111 is transmitted to the mobile monitoring device 100, while the random pairing code 2222 is the code paired with 1111. Based on this pair of codes, a communicative connection can be established.

Similarly, the second connection information includes at least one of a device identifier or a serial number of the mobile monitoring device 100; one of a communication channel identifier, an IP address, an MAC address, a service set identifier (SSID), WEP/WPA/WPA2/WPA3 protocol information, EAP information, which are determined by the mobile monitoring device 100; and a random pairing code which is generated by the mobile monitoring device 100. Wherein, the random pairing code which is generated by the mobile monitoring device 100 can be one code or a pair of codes. Wherein, the mobile monitoring device 100 generates one code means that the pairing codes between the mobile monitoring unit and the mobile monitoring device 100 are exactly the same, for example, the code is 1111. The mobile monitoring device 100 generates a pair of codes means that the mobile monitoring device 100 and the mobile monitoring unit have different pairing codes which have a corresponding relationship. For example, the random pairing codes generated by the mobile monitoring device 100 are 1111 and 2222 (for example, a pairing relationship list is established through an array), wherein the random pairing code 1111 is transmitted to the mobile monitoring unit, while the random pairing code 2222 is the code paired with 1111. Based on this pair of codes, a communicative connection can be established.

In an embodiment of this disclosure, in order to establish, based on the first connection information or the second connection information, the first communicative connection with the mobile monitoring unit, through the first wireless communication unit 130, the processor 120 is further configured to broadcast, through the first wireless communication unit 130, the first connection information or the second connection information, so as to enable the mobile monitoring unit to establish the first communicative connection with the mobile monitoring device 100, when the first connection information or the second connection information is detected by the mobile monitoring unit; or monitor information through the first wireless communication unit 130 and to establish the first communicative connection with the mobile monitoring unit, when the first connection information or the second connection information which is broadcasted by the mobile monitoring unit, is detected by the first wireless communication unit 130. In this embodiment, after the mobile monitoring device 100 and the mobile monitoring unit agreed on connection information (first connection information or second connection information), either one of the mobile monitoring device 100 and the mobile monitoring unit can broadcast the connection information, and accordingly, when the other one monitors the connection information, the communicative connection between them can be established. Generally, a device that broadcasts the connection information is called as a slave device, and a device that monitors the connection information is called as a master device.

In an embodiment of this disclosure, the mobile monitoring device 100 may further include a proximity-based communication unit (not shown), and the processor 120 is further configured to perform following operations: acquiring, from the near-end device, third connection information, in a contactless manner through the proximity-based communication unit, or transmitting, to the near-end device, fourth connection information, in a contactless manner through the proximity-based communication unit; and establishing, based on the third connection information or the fourth connection information, the second communicative connection with the near-end device through the first wireless communication unit 130; or acquiring, from the near-end device, third connection information, in a contactless manner through the proximity-based communication unit, or transmitting, to the near-end device, fourth connection information, in a contactless manner through the proximity-based communication unit; and establishing, based on the third connection information or the fourth connection information, the second communicative connection with the near-end device through the first wireless communication unit 130.

In this embodiment, the mobile monitoring device 100 includes at least one proximity-based communication unit. Based on the proximity-based communication unit, the processor 120 of the mobile monitoring device 100 can acquire the third connection information from the near-end device in a contactless manner. The third connection information is pairing information between the mobile monitoring device 100 and the near-end device. Alternatively, the processor 120 of the mobile monitoring device 100 can transmit the fourth connection information to the near-end device in a contactless manner. The fourth connection information is pairing information between the mobile monitoring device 100 and the near-end device. In this way, the mobile monitoring device 100 and the near-end device have the same pairing information or the pairing information with a corresponding relationship (i.e., the aforementioned third connection information or fourth connection information). Based on the pairing information, the aforementioned second communicative connection can be conveniently established between the mobile monitoring device 100 and the near-end device through the first wireless communication unit 130, so as to exchange data therebetween. The above pairing process does not require manual binding and pairing of patient information. Instead, the mobile monitoring device 100 and the near-end device are paired and connected through the agreed connection information (pairing information) between the two devices. In this way, the user only needs to move the mobile monitoring device 100 and the near-end device which serve the same patient close to each other, the pairing desired by the user can be achieved (that is, the user desires pairing between the two devices currently close to each other rather than pairing between other devices), which greatly simplifies the pairing process between different medical devices which serve the same patient, not only improves the user experience, but also minimizes medical accidents caused by manual operation errors.

In an embodiment of this disclosure, the third connection information includes at least one of: a device identifier (i.e., device ID) or a serial number (SN) of the near-end device; one of a communication channel identifier, an IP address, an MAC address, a service set identifier (SSID), Wired Equivalent Privacy (WEP) protocol information, Wi-Fi Protected Access (WPA) protocol information, WPA2 protocol information, WPA3 protocol information, Extensible Authentication Protocol (EAP) information, which are determined by the near-end device; and a random pairing code which is generated by the near-end device. Wherein, the random pairing code which is generated by the near-end device can be one code or a pair of codes. Wherein, the near-end device generates one code means that the pairing codes between the near-end device and the mobile monitoring device 100 are exactly the same, for example, the code is 1111. The near-end device generates a pair of codes means that the near-end device and the mobile monitoring device 100 have different pairing codes which have a corresponding relationship. For example, the random pairing codes generated by the near-end device are 1111 and 2222 (for example, a pairing relationship list is established through an array), wherein the random pairing code 1111 is transmitted to the mobile monitoring device 100, while the random pairing code 2222 is the code paired with 1111. Based on this pair of codes, a communicative connection can be established.

Similarly, the fourth connection information includes at least one of: a device identifier or a serial number of the mobile monitoring device 100; one of a communication channel identifier, an IP address, an MAC address, a service set identifier (SSID), WEP/WPA/WPA2/WPA3 protocol information, EAP information, which are determined by the mobile monitoring device 100; and a random pairing code which is generated by the mobile monitoring device 100. Wherein, the random pairing code which is generated by the mobile monitoring device 100 can be one code or a pair of codes. Wherein, the mobile monitoring device 100 generates one code means that the pairing codes between the near-end device and the mobile monitoring device 100 are exactly the same, for example, the code is 1111. The mobile monitoring device 100 generates a pair of codes means that the mobile monitoring device 100 and the near-end device have different pairing codes which have a corresponding relationship. For example, the random pairing codes generated by the mobile monitoring device 100 are 1111 and 2222 (for example, a pairing relationship list is established through an array), wherein the random pairing code 1111 is transmitted to the near-end device, while the random pairing code 2222 is the code paired with 1111. Based on this pair of codes, a communicative connection can be established.

In an embodiment of this disclosure, in order to establish, based on the third connection information or the fourth connection information, the second communicative connection with the near-end device through the first wireless communication unit 130, the processor 120 is further configured to broadcast, through the first wireless communication unit 130, the third connection information or the fourth connection information, so as to enable the near-end device to establish the second communicative connection with the mobile monitoring device 100, when the third connection information or the fourth connection information is detected by the near-end device; or monitor information through the first wireless communication unit 130 and to establish the second communicative connection with the near-end device, when the third connection information or the fourth connection information, which is broadcasted by the near-end device, is detected by the first wireless communication unit 130. In this embodiment, after the mobile monitoring device 100 and the near-end device agreed on connection information (third connection information or fourth connection information), either one of the mobile monitoring device 100 and the near-end device can broadcast the connection information, and accordingly, when the other one monitors the connection information, the communicative connection between them can be established. Generally, a device that broadcasts the connection information is called as a slave device, and a device that monitors the connection information is called as a master device.

The above exemplarily describes the mobile monitoring device according to the embodiment of this disclosure. Based on the above description, the mobile monitoring device according to the embodiment of this disclosure can communicate with another mobile monitoring unit on the human body and a near-end device apart from the human body based on the same wireless communication technology, thus reducing a complexity of wireless networking technology, saving power consumption, improving battery life, and also providing further possibilities for device miniaturization. In addition, the mobile monitoring device of this disclosure can also communicate with a far-end device based on another communication technology, so as to satisfy data transmission requirements in various scenarios.

A monitoring system provided in accordance with another aspect of this disclosure is described below.

FIG. 2 shows a structural block diagram of a monitoring system according to an embodiment of this disclosure. As shown in FIG. 2, the monitoring system 20 may include a wearable mobile monitoring device 100 and a mobile monitoring unit 200 which are worn on a human body, and a near-end device 300 and a far-end device 400 which are apart from the human body. The mobile monitoring device 100 is configured to monitor first monitoring data of the human body, the mobile monitoring unit 200 is configured to monitor second monitoring data of the human body, the near-end device 300 and the far-end device 400 are configured to acquire the first monitoring data and the third monitoring data. The mobile monitoring device 100 is further configured to perform following operations: receiving, from the mobile monitoring unit 200, the second monitoring data, through first communicative connection which is established with the mobile monitoring unit 200; transmitting, under a first preset condition to the near-end device 300 which is apart from the human body, the first monitoring data and the second monitoring data, through second communicative connection, which is established with the near-end device 300, wherein the second communicative connection and the first communicative connection are based on a same wireless communication technology, the near-end device 300 is located within a preset range; transmitting, under a second preset condition to the far-end device 400, the first monitoring data and the second monitoring data, through third communicative connection, which is established with the far-end device 400, wherein the third communicative connection and the first communicative connection are based on different wireless communication technologies, and the far-end device 400 is located outside the preset range.

In an embodiment of this disclosure, the monitoring system 20 includes a wearable mobile monitoring device 100 and a mobile monitoring unit 200 which are worn on the human body, a near-end device 300 and a far-end device 400 which are apart from the human body, wherein the mobile monitoring device 100 serves as a central node device, which transmits, to the near-end device 300 and/or the far-end device 400, the first monitoring data which is monitored by itself, and the second monitoring data which is acquired from the mobile monitoring unit 200 by itself. As the mobile monitoring device 100 communicates with the mobile monitoring unit 200, which is worn on the human body, through the first communicative connection, and communicates with the near-end device 300, which is apart from the human body, through the second communicative connection under the first preset condition, wherein the first communicative connection and the second communicative connection are based on the same wireless communication technology, that is, the mobile monitoring device 100 communicates with the mobile monitoring unit 200, which is worn on the human body, and the near-end device 300, which is apart from the human body, based on the same wireless communication technology, such that, in the entire monitoring system 20, the mobile monitoring device 100, the mobile monitoring unit 200 worn on the human body, and the near-end device 300 apart from the human body can complete their wireless network deployments within a same body area network, thus simplifying the complexity of network deployment. Moreover, since the mobile monitoring device 100 is a wearable device, it is usually worn on the human body to work. The mobile monitoring device 100 is close to the mobile monitoring unit worn on the human body (i.e., another wearable mobile monitoring devices 100, which is named as a mobile monitoring unit for distinguishing). The near-end device 300 is located within a preset range, that is, the near-end device 300 is a device which is relatively close to the mobile monitoring device 100. Therefore, the first communicative connection and the second communicative connection are both based on a technology related to near-field communication. Accordingly, in the monitoring system 20, the communication between the mobile monitoring device 100 and the mobile monitoring unit worn on the human body, as well as the communication between the mobile monitoring device 100 and the near-end device 300 apart from the human body under the first preset condition, are both through a wireless communication technology related to near-field communication, which can greatly reduce power consumption, thereby improving battery life, and also providing further possibilities for device miniaturization. In addition, in the monitoring system 20, the mobile monitoring device 100 communicates with the far-end device 400 through the third communicative connection under the second preset condition, and the first communicative connection (the second communicative connection) and the third communicative connection are based on different wireless communication technologies. As the far-end device 400 is located outside the preset range, that is, the far-end device 400 is a device far from the mobile monitoring device 100, the third communicative connection is based on a technology related to far-field communication. Therefore, in the monitoring system 20, the mobile monitoring device 100 can communicate with the far-end device 400, thereby satisfying the data transmission requirements in different scenarios.

In an embodiment of this disclosure, the aforementioned first preset condition may include: a communication quality for current communication between the mobile monitoring device 100 and the near-end device 300 through the second communicative connection, satisfies a preset standard. In this embodiment, when the communication quality for current communication between the mobile monitoring device 100 and the near-end device 300 through the second communicative connection, satisfies the preset standard, the mobile monitoring device 100 communicates with the near-end device 300 through the second communicative connection, so as to transmit, to the near-end device 300, the first monitoring data which is monitored by itself and the second monitoring data which is monitored by the mobile monitoring unit 200. Wherein, "satisfying the preset standard" can be understood as the mobile monitoring device 100 is capable of communicating with the near-end device 300 through the second communicative connection, or the communication quality between the mobile monitoring device 100 and the near-end device 300 is reliable. For example, it can be guaranteed that there is no packet loss in data transmitted from the mobile monitoring device 100 to the near-end device 300 through the second communicative connection, and each data transmission can receive a response which indicates that the data is successfully received, etc. Whenever the first preset condition is satisfied, the mobile monitoring device 100 can communicate with the near-end device 300 through the second communicative connection, so as to reduce power consumption and improve battery life, as much as possible.

In an embodiment of this disclosure, the aforementioned second preset condition may include: a communication quality for current communication between the mobile monitoring device 100 and the near-end device 300 through the second communicative connection, does not satisfy a preset standard. In this embodiment, when the communication quality of current communication between the mobile monitoring device 100 and the near-end device 300 through the second communicative connection, does not satisfy the preset standard, the mobile monitoring device 100 communicates with the far-end device 400 through the third communicative connection, so as to transmit, to the far-end device 400, the first monitoring data which is monitored by itself and the second monitoring data which is monitored by the mobile monitoring unit 200. Wherein, "does not satisfy the preset standard" can be understood as the mobile monitoring device 100 is not capable of communicating with the near-end device 300 through the second communicative connection, or the communication quality between the mobile monitoring device 100 and the near-end device 300 is not reliable. For example, there is a packet loss in data transmitted from the mobile monitoring device 100 to the near-end device 300 through the second communicative connection, or no response is received based on the data transmission, etc. Whenever the second preset condition is satisfied, the mobile monitoring device 100 can communicate with the far-end device 400 through the third communicative connection, so as to guarantee outgoing transmission of monitoring data.

In an embodiment of this disclosure, when transmitting the first monitoring data and the second monitoring data, the mobile monitoring device 100 can also be configured to: transmit, under a third preset condition, the first monitoring data and the second monitoring data, through the second communicative connection or the third communicative connection in a continuous transmission mode; or transmit under a preset condition which is other than the third present condition, the first monitoring data and the second monitoring data, through the second communicative connection or the third communicative connection in a continuous transmission mode or a discontinuous transmission mode; wherein the continuous transmission mode refers to transmitting data in multiple continuous transmission cycles, and the discontinuous transmission mode refers to transmitting data only in a portion of transmission cycle(s) of multiple continuous transmission cycles. In this embodiment, when the mobile monitoring device 100 transmits data to the near-end device 300 or the far-end device 400, it provides two data transmission modes, that is, the continuous transmission mode and the discontinuous transmission mode. The continuous transmission mode, as the name implies, is to transmit data continuously and uninterruptedly. For example, a transmission cycle (such as 1 second, etc., wherein the transmission cycle is a time period, which is required to transmit data once), is preset, then in each transmission cycle, the continuous transmission mode transmits data. The discontinuous transmission mode, as the name implies, is to transmit data discontinuously and intermittently. That is, for the preset transmission cycle, data is not transmitted in every transmission cycle, but data is transmitted some transmission cycle(s), and is not transmitted in other transmission cycle(s). Based on these two data transmission modes, under the third preset condition, the continuous transmission mode is used to transmit to the near-end device 300 or the far-end device 400 (transmitting to the near-end device 300 or the far-end device 400 depends on the first preset condition or second preset condition mentioned above), the first monitoring data, which is monitored by the mobile monitoring device 100 itself, and the second monitoring data, which is monitored by the mobile monitoring unit 200 (worn by the human body of the patient) and then acquired by the mobile monitoring device 100; while under the preset condition which is other than the third present condition, the discontinuous transmission mode is used to transmit to the near-end device 300 or the far-end device 400 (transmitting to the near-end device 300 or the far-end device 400 depends on the first preset condition or second preset condition mentioned above), the first monitoring data, which is monitored by the mobile monitoring device 100 itself, and the second monitoring data, which is monitored by the mobile monitoring unit 200 (worn by the human body of the patient) and then acquired by the mobile monitoring device 100. In such a way, power consumption can be saved.

In an embodiment of this disclosure, the aforementioned third preset condition may also include at least one of a condition which reflects a human health risk, a condition which reflects a parameter transmission mode, and a condition which reflects a user instruction. In this embodiment, the data transmission mode can be determined by combining at least one of the condition which reflects a human health risk, the condition which reflects a parameter transmission mode, and the condition which reflects a user instruction, so as to satisfy different application scenarios and/or user requirements. These three conditions are described below.

For the condition which reflects a human health risk, it can be understood that occurrence of such condition reflects that human health has a risk. Accordingly, when no condition which reflects a human health risk, exists, the discontinuous transmission mode is used to transmit data, so that the data transmission mode can be determined according to health and physiological requirements of the patient, which can save power consumption as much as possible while avoiding the occurrence of patient risk event and ensuring the life safety of patient. Of course, when no condition which reflects a human health risk, exists, the continuous transmission mode can also be used to transmit data, which can satisfy an application scenario, where the user does not care about battery life and pays more attention to monitoring.

In an embodiment of this disclosure, the aforementioned condition which reflects a human health risk may further include a monitoring time of the mobile monitoring device 100 for the human body, does not reach a preset time threshold; and/or, the first monitoring data and/or the second monitoring data indicate(s) that the human body is currently in a risk state. In this embodiment, a health risk situation of the human body is determined based on the monitoring time and/or the monitoring data. For example, for a patient who has undergone surgery, a period from when the surgery completes to a certain time period after the surgery (such as within 8 hours after surgery) is usually a high-risk period. The condition which reflects a human health risk, exists during this period, that is, during this period, the first monitoring data and the second monitoring data are transmitted in the continuous transmission mode, so as to ensure the life safety of patient. On the contrary, when the monitoring time for the human body reaches the preset time threshold (such as 8 hours have passed after the surgery), then the patient has usually passed the high-risk period and can be regarded as that the condition which reflects a human health risk, does not exist. That is, at this time, the first monitoring data and the second monitoring data are transmitted in the discontinuous transmission mode, so as to save power consumption. For another example, after the mobile monitoring device 100 acquires the first monitoring data and the second monitoring data, it can perform a simple analysis on the monitoring data, for example, determining whether the monitoring data is within a safety threshold range. If the monitoring data exceeds the safety threshold range, it indicates that the human body is currently in a risk state, and it is considered that the condition which reflects a human health risk exists at this time, that is, the continuous transmission mode is used to transmit the first monitoring data and the second monitoring data during this period to ensure the life safety of patient. Conversely, if the monitoring data is determined as within the safety threshold range, it indicates that the human body is not currently in a risk state, which means the condition which reflects a human health risk does not exist at this time, that is, the discontinuous transmission mode is used to transmit the first monitoring data and the second monitoring data during this period, so as to save power consumption.

For a condition which reflects a parameter transmission mode, it is easy to understand that the occurrence of this condition reflects a specific parameter transmission mode, so that a corresponding data transmission mode can be determined based on the parameter transmission mode reflected in this condition. For example, the aforementioned condition which reflects a parameter transmission mode may include the first monitoring data and the second monitoring data are transmitted through a first wireless communication unit of the mobile monitoring device 100, which unit includes a BLE communication module or an MBAN communication module. In this embodiment, since the monitoring data is transmitted through the BLE communication module or the MBAN communication module, and as described above, a wireless communication technology supported by the BLE communication module or the MBAN communication module is a technology related to near-field communication. As the power consumption of the technology related to near-field communication is low, so in this case, the continuous transmission mode can be used to transmit, to the near-end device 300, the first monitoring data, which is monitored by the mobile monitoring device 100 itself, and the second monitoring data, which is monitored by the mobile monitoring unit 200 (worn by the human body of the patient) and then acquired by the mobile monitoring device 100. That is, when the mobile monitoring device 100, transmits, through the first wireless communication unit, the monitoring data to the near-end device 300, even if the continuous transmission mode is adopted, more complete monitoring data records can be ensured at the near-end device 300 without consuming too much power, which is conducive to real-time viewing, monitoring or reviewing of the patient condition at the near-end device 300. On the contrary, if the first monitoring data and the second monitoring data are transmitted not through the first wireless communication unit, but through a second wireless communication unit, which includes at least one of a WIFI wireless communication module, a WMTS wireless communication and a cellular network communication module, this can be considered as the preset condition which is other than the third preset condition, and as described above. However, a wireless communication technology supported by the WIFI wireless communication module, the WMTS wireless communication or the cellular network communication module is a technology related to far-field communication. Since the power consumption of the technology related to far-field communication is high, in this case, the discontinuous transmission mode can be used to transmit, to the far-end device 400, the first monitoring data, which is monitored by the mobile monitoring device 100 itself, and the second monitoring data, which is monitored by the mobile monitoring unit 200 (worn by the human body of the patient) and then acquired by the mobile monitoring device 100, so as to save power consumption.

For the condition which reflects a user instruction, it is also easy to understand that the condition reflects a user requirement, so that the corresponding data transmission mode can be determined according to a user instruction in this condition. For example, the aforementioned condition which reflects a user instruction may include a user instruction, which instructs to transmit the first monitoring data and the second monitoring data in the continuous transmission mode, is received by the mobile monitoring device 100. In this embodiment, when no condition which reflects a human health risk, exists, if a user instruction, which instructs to transmit the first monitoring data and the second monitoring data in the continuous transmission mode, is received, the mobile monitoring device 100 transmits, to the near-end device 300 or the far-end device 400 in the continuous transmission mode based on the user instruction, the first monitoring data, which is monitored by the mobile monitoring device 100 itself, and the second monitoring data, which is monitored by the mobile monitoring unit 200 (worn by the human body of the patient) and then acquired by the mobile monitoring device 100, so as to satisfy the user requirement. This embodiment is generally applied to an application scenario where the user does not care about battery life but is more concerned about monitoring.

Furthermore, when at least two conditions are combined to determine the data transmission mode, a priority of each condition may be preset. For example, among the above three conditions, the condition which reflects a human health risk has a highest priority. In this way, when the condition which reflects a human health risk, exists, the continuous transmission mode is used to transmit data regardless of other conditions; when the condition which reflects a human health risk, does not exist, the transmission mode of the monitoring data can be determined in combination with the condition which reflects a parameter transmission mode and/or the condition which reflects a user instruction. In this way, different communication scenarios and/or different user requirements can be satisfied, while ensuring life safety of patient.

In an embodiment of this disclosure, the mobile monitoring device 100 may be further configured to store, the first monitoring data and the second monitoring data, when the second communicative connection between the mobile monitoring device 100 and the near-end device 300 is disconnected. In this embodiment, the mobile monitoring device 100 itself may have a storage function for the monitoring data. When the mobile monitoring device 100 is unable to transmit to the near-end device 300, the first monitoring data which is acquired through its own monitoring and the second monitoring data which is acquired from another mobile monitoring unit 200 worn on the human body, the mobile monitoring device 100 can use its own memory to store the first monitoring data and the second monitoring data, so as to ensure the complete preservation of the monitoring data. Further, the mobile monitoring device 100 may also be configured to transmit, to the near-end device 300, the first monitoring data and the second monitoring data which are stored, through the second communicative connection between the mobile monitoring device 100 and the near-end device 300, when the second communicative connection is restored. In this embodiment, when the second communicative connection between the mobile monitoring device 100 and the near-end device 300 is restored, the mobile monitoring device 100 may transmit, to the near-end device 300, the first monitoring data and the second monitoring data, which are stored during a disconnection of the second communicative connection, so as to guarantee the integrity of monitoring data at the near-end device 300.

In an embodiment of this disclosure, the mobile monitoring device 100 may be further configured to display the first monitoring data and/or the second monitoring data. In this embodiment, the mobile monitoring device 100 itself can display the first monitoring data which is acquired through its own monitoring and the second monitoring data which is acquired from another mobile monitoring unit worn on the human body, so as to facilitate the patient itself to view.

In an embodiment of this disclosure, the first communicative connection mentioned above may include BLE wireless communicative connection or MBAN wireless communicative connection; the second communicative connection mentioned above may include BLE wireless communicative connection or MBAN wireless communicative connection.

In an embodiment of this disclosure, the third communicative connection mentioned above may include at least one of WIFI wireless communicative connection, WMTS wireless communicative connection, and cellular communicative connection.

In an embodiment of this disclosure, the mobile monitoring device 100 in the monitoring system 20 may be a monitoring device that can be worn on the human body and acquire a signal which characterizes at least one vital sign parameter of the human body, such as a monitoring device worn on the wrist to monitor blood oxygen, and so on. The mobile monitoring unit 200 in the monitoring system 20 can be another monitoring device worn on the human body to acquire a signal which characterizes at least one vital sign parameter of the human body, such as a monitoring device worn on the neck and chest of the human body for monitoring an ECG signal, a monitoring device worn on upper arms of the human body for monitoring blood pressure, etc. In an embodiment of this disclosure, the aforementioned near-end device 300 in the monitoring system 20 may be a medical device (such as a bedside monitor, ultrasound device, etc.) in the same ward as the mobile monitoring device 100, or may be a non-medical electronic device (such as mobile phones, computers, etc.), which is close to the mobile monitoring device 100. In an embodiment of this disclosure, the aforementioned far-end device 400 in the monitoring system 20 can be various systems in the hospital where the patient is located, such as a central station at a nurse desk, etc., or other non-medical remote electronic devices, such as a client of telemedicine service, etc.

In an embodiment of this disclosure, when the mobile monitoring device 100 transmits the first monitoring data and the second monitoring data to the far-end device 400, it may directly or indirectly transmit the data to the far-end device 400. The indirect transmission to the far-end device 400 may refer to that the mobile monitoring device 100 establishes the aforementioned third communicative connection with a forwarding device, transmits the first monitoring data and the second monitoring data to the forwarding device based on the third communicative connection, and then uses the forwarding device to forward the first monitoring data and the second monitoring data to the far-end device 400. Wherein, when the third communicative connection includes WIFI communicative connection and/or WMTS communicative connection, the forwarding device may be a WIFI wireless access point (AP for short) device and/or a WMTS AP device. When the third communicative connection includes cellular communicative connection, the forwarding device may be a base station.

In an embodiment of this disclosure, the mobile monitoring device 100 may be further configured to perform following operations: acquiring, from the mobile monitoring unit 200, first connection information, in a contactless manner, or transmitting, to the mobile monitoring unit 200, second connection information, in a contactless manner; and establishing, based on the first connection information or the second connection information, the first communicative connection with the mobile monitoring unit 200; and/or acquiring, from the near-end device 300, third connection information, in the contactless manner, or transmitting, to the near-end device 300, fourth connection information, in a contactless manner; and establishing, based on the third connection information or the fourth connection information, the second communicative connection with the near-end device 300.

In this embodiment, the mobile monitoring device 100 can acquire the first connection information from the mobile monitoring unit 200 in a contactless manner. The first connection information is pairing information between the mobile monitoring device 100 and the mobile monitoring unit 200. Alternatively, the mobile monitoring device 100 can transmit the second connection information to the mobile monitoring unit 200 in a contactless manner, and the second connection information is pairing information between the mobile monitoring device 100 and the mobile monitoring unit 200. In this way, the mobile monitoring device 100 and the mobile monitoring unit 200 have the same pairing information or the pairing information with a corresponding relationship (i.e., the aforementioned first connection information or the second connection information). Based on the pairing information, the first communicative connection mentioned above can be conveniently established between the mobile monitoring unit 200 and the mobile monitoring device 100, so as to exchange data therebetween. The above pairing process does not require manual binding and pairing of patient information. Instead, the mobile monitoring device 100 and the mobile monitoring unit 200 are paired and connected through the agreed connection information (pairing information) between the mobile monitoring device 100 and the mobile monitoring unit 200. In this way, the user only needs to move the mobile monitoring device 100 and the mobile monitoring unit 200 which serve the same patient close to each other, the pairing desired by the user can be achieved (that is, the user desires pairing between the mobile monitoring device 100 and the mobile monitoring unit 200 currently close to each other rather than pairing between other devices), which greatly simplifies the pairing process between different medical devices which serve the same patient, not only improves the user experience, but also minimizes medical accidents caused by manual operation errors.

In an embodiment of this disclosure, the first connection information includes at least one of: a device identifier (i.e., device ID) or a serial number (SN) of the mobile monitoring unit 200; one of a communication channel identifier, an IP address, an MAC address, a service set identifier (SSID), Wired Equivalent Privacy (WEP) protocol information, Wi-Fi Protected Access (WPA) protocol information, WPA2 protocol information, WPA3 protocol information, Extensible Authentication Protocol (EAP) information, which are determined by the mobile monitoring unit 200; and a random pairing code which is generated by the mobile monitoring unit 200. Wherein, the random pairing code which is generated by the mobile monitoring unit 200 can be one code or a pair of codes. Wherein, the mobile monitoring unit 200 generates one code means that the pairing codes between the mobile monitoring unit 200 and the mobile monitoring device 100 are exactly the same, for example, the code is 1111. The mobile monitoring unit 200 generates a pair of codes means that the mobile monitoring unit 200 and the mobile monitoring device 100 have different pairing codes which have a corresponding relationship. For example, the random pairing codes generated by the mobile monitoring unit 200 are 1111 and 2222 (for example, a pairing relationship list is established through an array), wherein the random pairing code 1111 is transmitted to the mobile monitoring device 100, while the random pairing code 2222 is the code paired with 1111. Based on this pair of codes, a communicative connection can be established.

Similarly, the second connection information includes at least one of a device identifier or a serial number of the mobile monitoring device 100; one of a communication channel identifier, an IP address, an MAC address, a service set identifier (SSID), WEP/WPA/WPA2/WPA3 protocol information, EAP information, which are determined by the mobile monitoring device 100; and a random pairing code which is generated by the mobile monitoring device 100. Wherein, the random pairing code which is generated by the mobile monitoring device 100 can be one code or a pair of codes. Wherein, the mobile monitoring device 100 generates one code means that the pairing codes between the mobile monitoring unit 200 and the mobile monitoring device 100 are exactly the same, for example, the code is 1111. The mobile monitoring device 100 generates a pair of codes means that the mobile monitoring device 100 and the mobile monitoring unit 200 have different pairing codes which have a corresponding relationship. For example, the random pairing codes generated by the mobile monitoring device 100 are 1111 and 2222 (for example, a pairing relationship list is established through an array), wherein the random pairing code 1111 is transmitted to the mobile monitoring unit 200, while the random pairing code 2222 is the code paired with 1111. Based on this pair of codes, a communicative connection can be established.

In an embodiment of this disclosure, in order to establish, based on the first connection information or the second connection information, the first communicative connection with the mobile monitoring unit 200, the mobile monitoring device 100 is further configured to broadcast the first connection information or the second connection information, so as to enable the mobile monitoring unit 200 to establish the first communicative connection with the mobile monitoring device 100, when the first connection information or the second connection information is detected by the mobile monitoring unit 200; or to monitor information and to establish the first communicative connection with the mobile monitoring unit 200, when the first connection information or the second connection information which is broadcasted by the mobile monitoring unit 200, is detected by the mobile monitoring device 100. In this embodiment, after the mobile monitoring device 100 and the mobile monitoring unit 200 agreed on connection information (first connection information or second connection information), either one of the mobile monitoring device 100 and the mobile monitoring unit 200 can broadcast the connection information, and accordingly, when the other one monitors the connection information, the communicative connection between them can be established. Generally, a device that broadcasts the connection information is called as a slave device, and a device that monitors the connection information is called as a master device.

In an embodiment of this disclosure, the mobile monitoring device 100 may be further configured to perform following operations: acquiring, from the near-end device 300, third connection information, in a contactless manner, or transmitting, to the near-end device 300, fourth connection information, in a contactless manner; establishing, based on the third connection information or the fourth connection information, the second communicative connection with the near-end device 300; or acquiring, from the near-end device 300, third connection information, in a contactless manner, or transmitting, to the near-end device 300, fourth connection information, in a contactless manner; and establishing, based on the third connection information or the fourth connection information, the second communicative connection with the near-end device 300.

In this embodiment, the mobile monitoring device 100 can acquire the third connection information from the near-end device 300 in a contactless manner. The third connection information is pairing information between the mobile monitoring device 100 and the near-end device 300. Alternatively, the mobile monitoring device 100 can transmit the fourth connection information to the near-end device 300 in a contactless manner. The fourth connection information is pairing information between the mobile monitoring device 100 and the near-end device 300. In this way, the mobile monitoring device 100 and the near-end device 300 have the same pairing information or the pairing information with a corresponding relationship (i.e., the aforementioned third connection information or fourth connection information). Based on the pairing information, the aforementioned second communicative connection can be conveniently established between the mobile monitoring device 100 and the near-end device 300, so as to exchange data therebetween. The above pairing process does not require manual binding and pairing of patient information. Instead, the mobile monitoring device 100 and the near-end device 300 are paired and connected through the agreed connection information (pairing information) between the two devices. In this way, the user only needs to move the mobile monitoring device 100 and the near-end device 300 which serve the same patient close to each other, the pairing desired by the user can be achieved (that is, the user desires pairing between the two devices currently close to each other rather than pairing between other devices), which greatly simplifies the pairing process between different medical devices which serve the same patient, not only improves the user experience, but also minimizes medical accidents caused by manual operation errors.

In an embodiment of this disclosure, the third connection information includes at least one of: a device identifier (i.e., device ID) or a serial number (SN) of the near-end device 300; one of a communication channel identifier, an IP address, an MAC address, a service set identifier (SSID), Wired Equivalent Privacy (WEP) protocol information, Wi-Fi Protected Access (WPA) protocol information, WPA2 protocol information, WPA3 protocol information, Extensible Authentication Protocol (EAP) information, which are determined by the near-end device 300; and a random pairing code which is generated by the near-end device 300. Wherein, the random pairing code which is generated by the near-end device 300 can be one code or a pair of codes. Wherein, the near-end device 300 generates one code means that the pairing codes between the near-end device 300 and the mobile monitoring device 100 are exactly the same, for example, the code is 1111. The near-end device 300 generates a pair of codes means that the near-end device 300 and the mobile monitoring device 100 have different pairing codes which have a corresponding relationship. For example, the random pairing codes generated by the near-end device 300 are 1111 and 2222 (for example, a pairing relationship list is established through an array), wherein the random pairing code 1111 is transmitted to the mobile monitoring device 100, while the random pairing code 2222 is the code paired with 1111. Based on this pair of codes, a communicative connection can be established.

Similarly, the fourth connection information includes at least one of: a device identifier or a serial number (SN) of the mobile monitoring device 100; one of a communication channel identifier, an IP address, an MAC address, a service set identifier (SSID), WEP/WPA/WPA2/WPA3 protocol information, EAP information, which are determined by the mobile monitoring device 100; and a random pairing code which is generated by the mobile monitoring device 100. Wherein, the random pairing code which is generated by the mobile monitoring device 100 can be one code or a pair of codes. Wherein, the mobile monitoring device 100 generates one code means that the pairing codes between the near-end device 300 and the mobile monitoring device 100 are exactly the same, for example, the code is 1111. The mobile monitoring device 100 generates a pair of codes means that the mobile monitoring device 100 and the near-end device 300 have different pairing codes which have a corresponding relationship. For example, the random pairing codes generated by the mobile monitoring device 100 are 1111 and 2222 (for example, a pairing relationship list is established through an array), wherein the random pairing code 1111 is transmitted to the near-end device 300, while the random pairing code 2222 is the code paired with 1111. Based on this pair of codes, a communicative connection can be established.

In an embodiment of this disclosure, in order to establish, based on the third connection information or the fourth connection information, the second communicative connection with the near-end device 300, the mobile monitoring device 100 is further configured to broadcast the third connection information or the fourth connection information, so as to enable the near-end device 300 to establish the second communicative connection with the mobile monitoring device 100, when the third connection information or the fourth connection information is detected by the near-end device 300; or to monitor information and to establish the second communicative connection with the near-end device 300, when the third connection information or the fourth connection information, which is broadcasted by the near-end device 300, is detected by the mobile monitoring device 100. In this embodiment, after the mobile monitoring device 100 and the near-end device 300 agreed on connection information (third connection information or fourth connection information), either one of the mobile monitoring device 100 and the near-end device 300 can broadcast the connection information, and accordingly, when the other one monitors the connection information, the communicative connection between them can be established. Generally, a device that broadcasts the connection information is called as a slave device, and a device that monitors the connection information is called as a master device.

The above exemplarily describes the monitoring system according to the embodiment of this disclosure. Based on the above description, the monitoring system according to the embodiment of this disclosure uses a wearable mobile monitoring device as a central node device for wireless networking, which central node device can communicate with another mobile monitoring unit which is worn on the human body and a near-end device which is apart from the human body, based on the same wireless communication technology, thus reducing a complexity of wireless networking technology, saving power consumption, improving battery life, and also providing further possibilities for device miniaturization. In addition, the mobile monitoring device can also communicate with a far-end device based on another communication technology, so as to satisfy data transmission requirements in various scenarios.

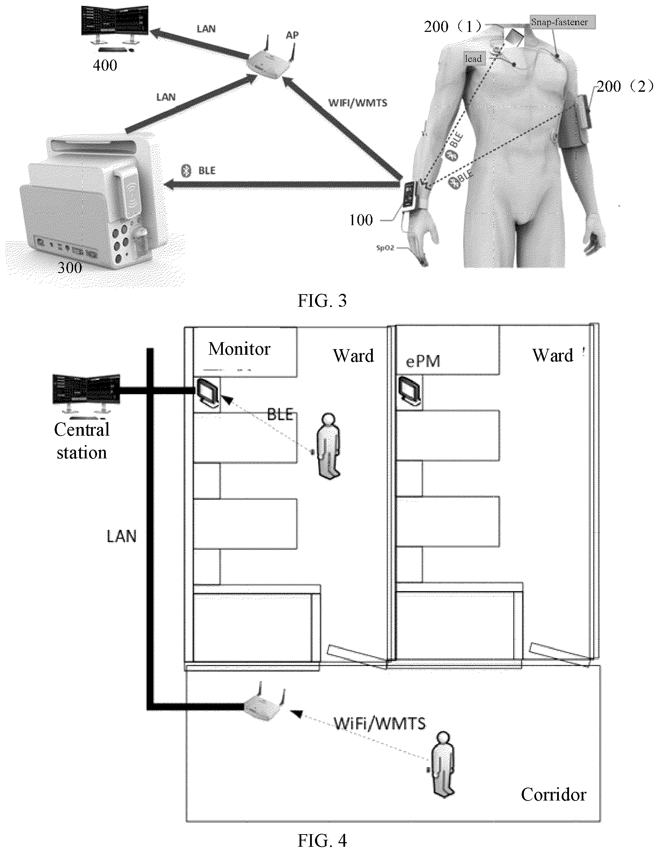

FIG. 3 shows an exemplary diagram of a wireless topology of a monitoring system according to an embodiment of this disclosure. As shown in FIG. 3, the monitoring system 30 includes a wearable mobile monitoring device 100 and mobile monitoring units 200 (1) and 200 (2) which are worn on the human body, and a near-end device 300 and a far-end device 400, which are apart from the human body. As an example, the mobile monitoring device 100 is a monitoring device worn on the wrist for monitoring blood oxygen (SPO2), the mobile monitoring unit 200(1) is shown as a monitoring device worn on the neck and chest of the human body for monitoring an ECG signal (including electrode pads, lead wires, and snaps connecting the electrode pads and lead wires); the mobile monitoring unit 200(2) is shown as a monitoring device worn on upper arms of the human body for monitoring blood pressure; the near-end device 300 is shown as a monitor; the far-end device 400 is shown as a central station. The mobile monitoring device 100 is configured to monitor and acquire blood oxygen data of the human body. The mobile monitoring unit 200(1) is configured to monitor and acquire electrocardiogram data of the human body, while the mobile monitoring unit 200(2) is configured to monitor and acquire blood pressure data of the human body. The terminal device 300 and the far-end device 400 are configured to acquire the blood oxygen data, electrocardiogram data and blood pressure data of the human body. The mobile monitoring device 100 is further configured to perform following operations: respectively receiving, from the mobile monitoring units 200(1) and 200(2), the ECG data and the blood pressure data, through BLE communicative connection, which is established with the mobile monitoring units 200(1) and 200(2); transmitting, under a first preset condition to the monitor which is apart from the human body, the blood oxygen data, the ECG data and the blood pressure data, through the BLE communicative connection, which is established with the monitor; transmitting, under second first preset condition to an AP device, the blood oxygen data, the ECG data and the blood pressure data, through WIFI communicative connection or WMTS communicative connection, which is established with the AP device; and then forwarding, to the central station, the blood oxygen data, the ECG data and the blood pressure data, through the AP device (for example, via a local area network LAN). In addition, the monitor can also transmit the blood oxygen data, ECG data and blood pressure data to the AP device through the LAN, and then the AP device forwards these data to the central station. The monitoring system 30 can be mainly used for monitoring sub-critically ill patients.

The monitoring system 30 uses a wearable mobile monitoring device as a central node device for wireless networking, which central node device can communicate with another mobile monitoring unit which is worn on the human body and a monitor which is apart from the human body, based on the same wireless communication technology, thus reducing a complexity of wireless networking technology, saving power consumption, improving battery life, and also providing further possibilities for device miniaturization. In addition, the mobile monitoring device can also communicate with the central station based on another communication technology, so as to satisfy data transmission requirements in various scenarios.

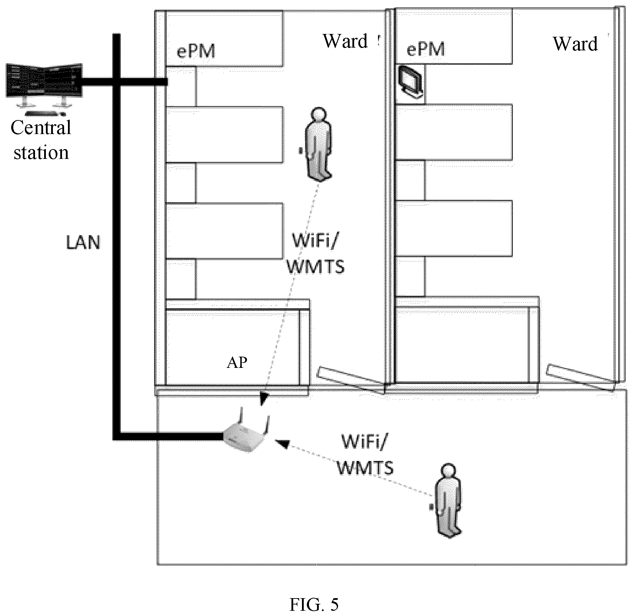

The data transmission diagram of the monitoring system shown in FIG. 3 and its deformation is further described below in conjunction with FIGS. 4-5. FIG. 4 shows a scene when a bedside monitor exists, while FIG. 5 shows a scene when a bedside monitor does not exist. The mobile monitoring device can transmit data in different ways depending on location of patient and existence of bedside monitor.

As shown in FIG. 4, when a bedside monitor exists and the patient is in the ward, the mobile monitoring device takes advantage of short communication distance and low power consumption of Bluetooth, so as to allow the monitor to join the body LAN, the mobile monitoring device uses a BLE communication technology to transmit the blood oxygen data, ECG data, and blood pressure data to the bedside monitor. The bedside monitor can transmit data to the central station via wireless LAN or wired LAN. After the patient leaves the ward, the monitor disconnects from the body LAN, and the mobile monitoring device transmits the blood oxygen data, ECG data, and blood pressure data to the WIFI/WMTS AP device through WIFI/WMTS technology, and the AP device forwards theses data to the central station. In this topology, if no central station exists, or the network between the monitor and the central station is disconnected, the monitor does not need to transmit data to the central station.

As shown in FIG. 5, when no monitor exists next to the bed, no matter whether the patient is in the ward or outside the ward, the mobile monitoring device transmits the data to the WIFI/WMTS AP device through WIFI/WMTS technology, and then the AP device forwards the data to the central station.

In the network topology shown in FIGS. 4 and 5, when the patient is bedridden or has physiological abnormalities after surgery, the system works in a continuous transmission mode, and enters into a discontinuous transmission mode, if the risk is determined to be relatively low. In the discontinuous transmission mode, if the mobile monitoring device detects a physiological abnormality of the patient, it can switch the discontinuous transmission mode to the continuous transmission mode. Alternatively, if the user thinks the battery life is not important, the discontinuous transmission mode can be changed the continuous transmission mode, according to a user instruction. In addition, when the mobile monitoring device and the monitor are in low-power BLE connection, the data transmitted at this time is continuous (for example, data is transmitted every second for 2 minutes), which is the continuous transmission mode mentioned above. When the mobile monitoring device directly transmits data to the AP device, considering the high-power consumption of far-field communication, a regular discontinuous transmission mode is provided (for example, physiological data is transmitted in only 15 seconds of each 2 minutes), which is the discontinuous transmission mentioned above, so as to satisfy the corresponding power consumption requirement.

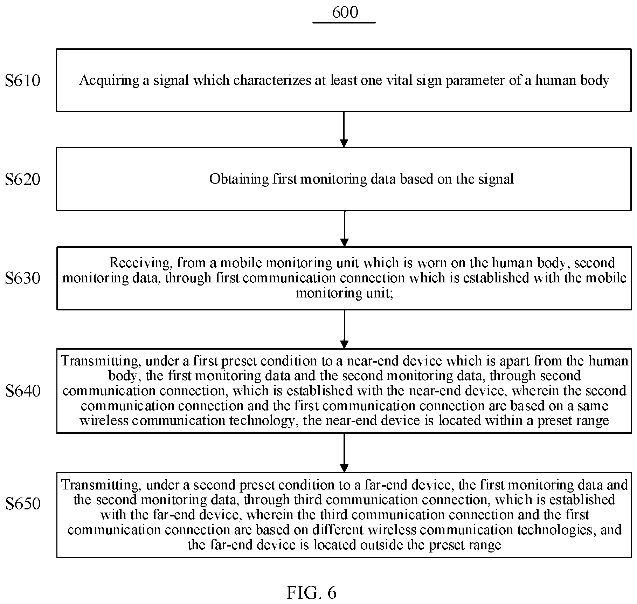

The following describes a data transmission method 600 provided according to another aspect of this disclosure in conjunction with FIG. 6. This method 600 is applied to the wearable mobile monitoring device described above. The data transmission process of the wearable mobile monitoring device has been described in detail above. Those skilled in the art can understand the specific details of the data transmission method 600 based on the foregoing description. For the sake of brevity, only some main steps of the data transmission method 600 are described here.

As shown in FIG. 6, the data transmission method 600 may include the following steps.

In step S610, a signal, which characterizes at least one vital sign parameter of a human body, is acquired.

In step S620, first monitoring data is obtained based on the signal.

In step S630, second monitoring data is received from a mobile monitoring unit which is worn on the human body, through first communicative connection, which is established between the mobile monitoring device and the mobile monitoring unit.

In step S640, under a first preset condition, the first monitoring data and the second monitoring data are transmitted to a near-end device which is apart from the human body, through second communicative connection, which connection is established with the near-end device, wherein the second communicative connection and the first communicative connection are based on a same wireless communication technology, the near-end device is located within a preset range.

In step S650, under a second preset condition, the first monitoring data and the second monitoring data are transmitted to a far-end device which is apart from the human body, through third communicative connection, which connection is established with the far-end device, wherein the third communicative connection and the first communicative connection are based on different wireless communication technologies, and the far-end device is located outside the preset range.

In an embodiment of this disclosure, the first preset condition includes: a communication quality for current communication between the mobile monitoring device and the near-end device through the second communicative connection, satisfies a preset standard.

In an embodiment of this disclosure, the second preset condition includes: a communication quality for current communication between the mobile monitoring device and the near-end device through the second communicative connection, does not satisfy a preset standard.

In an embodiment of this disclosure, transmitting the first monitoring data and the second monitoring data, includes: transmitting, under a third preset condition, the first monitoring data and the second monitoring data, through the second communicative connection or the third communicative connection in a continuous transmission mode; or transmitting, under a preset condition which is other than the third present condition, the first monitoring data and the second monitoring data, through the second communicative connection or the third communicative connection in a continuous transmission mode or a discontinuous transmission mode; wherein the continuous transmission mode refers to transmitting data in multiple continuous transmission cycles, and the discontinuous transmission mode refers to transmitting data only in a portion of transmission cycle(s) of multiple continuous transmission cycles.

In an embodiment of this disclosure, the third preset condition further includes at least one of: a condition which reflects a human health risk, a condition which reflects a parameter transmission mode, and a condition which reflects a user instruction.

In an embodiment of this disclosure, the condition which reflects a human health risk include: a monitoring time of the mobile monitoring device for the human body, does not reach a preset time threshold; and/or, the first monitoring data and/or the second monitoring data indicate(s) that the human body is currently in a risk state.

In an embodiment of this disclosure, the condition which reflects a user instruction includes a user instruction, which instructs to transmit the first monitoring data and the second monitoring data in the continuous transmission mode, is received by the mobile monitoring device.

In an embodiment of this disclosure, the condition which reflects a parameter transmission mode, includes the first monitoring data and the second monitoring data are transmitted through a first wireless communication unit of the mobile monitoring device, wherein the first wireless communication unit includes a Bluetooth communication module or an MBAN communication module.

In an embodiment of this disclosure, the first communicative connection includes Bluetooth communicative connection or Medical Body Area Network (MBAN) communicative connection.

In an embodiment of this disclosure, the third communicative connection includes at least one of WIFI communicative connection, WMTS communicative connection, and cellular network communicative connection.

In an embodiment of this disclosure, the method 600 may further include: storing the first monitoring data and the second monitoring data, when the second communicative connection with the near-end device is disconnected.

In an embodiment of this disclosure, the method 600 may further include: transmitting, to the near-end device, the first monitoring data and the second monitoring data which are stored, through the second communicative connection which is with the near-end device, when the second communicative connection is restored.

In an embodiment of this disclosure, the method 600 may further include: displaying the first monitoring data and/or the second monitoring data.

In an embodiment of this disclosure, the near-end device includes a monitor in a same ward as the mobile monitoring device, and the far-end device includes a central station.

In an embodiment of this disclosure, the method 600 further includes: acquiring, from the mobile monitoring unit, first connection information, in a contactless manner, or transmitting, to the mobile monitoring unit, second connection information, in a contactless manner; and establishing, based on the first connection information or the second connection information, the first communicative connection with the mobile monitoring unit; and/or acquiring, from the near-end device, third connection information, in a contactless manner, or transmitting, to the near-end device, fourth connection information, in a contactless manner; establishing, based on the third connection information or the fourth connection information, the second communicative connection with the near-end device.

In an embodiment of this disclosure, establishing, based on the first connection information or the second connection information, the first communicative connection with the mobile monitoring unit, includes: broadcasting the first connection information or the second connection information, so as to enable the mobile monitoring unit to establish the first communicative connection with the mobile monitoring device, when the first connection information or the second connection information is detected by the mobile monitoring unit; or monitoring information, and establishing the first communicative connection with the mobile monitoring unit, when the first connection information or the second connection information which is broadcasted by the mobile monitoring unit, is detected; establishing, based on the third connection information or the fourth connection information, the second communicative connection with the near-end device, includes: broadcasting the third connection information or the fourth connection information, so as to enable the near-end device to establish the second communicative connection with the mobile monitoring device, when the third connection information or the fourth connection information is detected by the near-end device; or monitoring information and establishing the second communicative connection with the near-end device, when the third connection information or the fourth connection information which is broadcasted by the near-end device, is detected.

In an embodiment of this disclosure, the first connection information includes at least one of a device identifier or a serial number of the mobile monitoring unit; one of a communication channel identifier, an IP address, an MAC address, a service set identifier, WEP/WPA/WPA2/WPA3 protocol information, EAP information, which are determined by the mobile monitoring unit; and a random pairing code which is generated by the mobile monitoring unit; the second connection information and the fourth connection information include at least one of a device identifier or a serial number of the mobile monitoring device; one of a communication channel identifier, an IP address, an MAC address, a service set identifier, WEP/WPA/WPA2/WPA3 protocol information, PARAMETER DATA FOR protocol information, which are determined by the mobile monitoring device; and a random pairing code which is generated by the mobile monitoring device; the third connection information includes at least one of: a device identifier or a serial number of the near-end device; one of a communication channel identifier, an IP address, an MAC address, a service set identifier, WEP/WPA/WPA2/WPA3 protocol information, EAP information, which are determined by the near-end device; and a random pairing code which is generated by the near-end device.

Based on the above description, the mobile monitoring device in the data transmission method according to the embodiment of this disclosure can communicate with another mobile monitoring unit worn on the human body and a near-end device apart from the human body based on the same wireless communication technology, thus reducing a complexity of wireless networking technology, saving power consumption, improving battery life, and also providing further possibilities for device miniaturization. In addition, the mobile monitoring device in the data transmission method according to the embodiment of this disclosure can also communicate with a far-end device based on another communication technology, so as to satisfy data transmission requirements in various scenarios.

In addition, according to the embodiment of this disclosure, a storage medium is also provided, and program instructions are stored on the storage medium. When the program instructions are run by a computer or processor, they are configured to execute corresponding steps of the data transmission method of the embodiment of this disclosure. The storage medium may include, for example, a memory card of a smartphone, a storage component of a tablet computer, a hard disk of a personal computer, a read-only memory (ROM), an erasable programmable read-only memory (EPROM), a portable compact disk read-only memory (CD-ROM), USB memory, or any combination of the above storage media. The computer-readable storage medium may be any combination of one or more computer-readable storage media.

Although the exemplary embodiments are described here with reference to the accompanying drawings, it should be understood that the aforementioned exemplary embodiments are only illustrative and are not intended to limit the scope of this disclosure. Ordinary technical personnel in this field can make various changes and modifications without deviating from the scope and spirit of this disclosure. All these changes and modifications are intended to be included within the scope of this disclosure as claimed in the attached claims.

Ordinary technical personnel in this field can realize that the units and algorithm steps described in combination with this disclosure can be implemented in electronic hardware, or a combination of computer software and electronic hardware. Whether these functions are executed in hardware or software depends on the specific application and design constraints of the technical solution. Professional technicians may use different methods to implement the described functions for each specific application, but such implementation should not be considered beyond the scope of this disclosure.

In the several embodiments provided in this disclosure, it should be understood that the disclosed devices and methods can be implemented in other ways. For example, the device embodiments described above are only schematic. For example, the division of the units is only a logical functional division, and there may be other division methods in actual implementations. For example, multiple units or components can be combined or integrated into another device, or some characteristics can be ignored or not executed.

The disclosure provided here provides a large number of specific details. However, it can be understood that the embodiments of this disclosure can be practiced without these specific details. In some examples, well-known methods, structures, and techniques are not shown in detail to avoid blurring the understanding of this disclosure.

Similarly, it should be understood that in order to streamline this disclosure and assist in understanding one or more of the various aspects of this disclosure, in the description of exemplary embodiments of this disclosure, the various characteristics of this disclosure are sometimes grouped together into a single embodiment, diagram, or description thereof. However, the method of this disclosure should not be interpreted as reflecting the intention that the claimed protection of this disclosure requires more characteristics than those explicitly recorded in each claim. More precisely, as reflected in the corresponding claims, the inventive point is that the corresponding technical problem can be solved with less than all characteristics of a single disclosed embodiment. Therefore, the claims following the specific implementation method are explicitly incorporated into the specific implementation method, where each claim itself serves as a separate embodiment of this disclosure.

Ordinary technical personnel in this field can understand that, in addition to mutual exclusion between characteristics, any combination can be configured to combine all characteristics disclosed in this disclosure (including accompanying claims, abstract, and drawings), as well as all processes or units of any method or device so disclosed. Unless otherwise explicitly stated, each characteristic disclosed in this disclosure (including accompanying claims, abstract, and accompanying drawings) may be replaced by alternative characteristics that provide the same, equivalent, or similar purpose.

In addition, Ordinary technical personnel in this field can understand that although some embodiments described herein include certain characteristics rather than other characteristics included in other embodiments, the combination of characteristics of different embodiments means that they are within the scope of this disclosure and form different embodiments. For example, in the claims, any one of the claimed embodiments can be used in any combination.

The various component embodiments of this disclosure can be implemented in hardware, software modules running on one or more processors, or a combination thereof. Ordinary technical personnel in this field should understand that a microprocessor or digital signal processor (DSP) can be used in practice to implement some or all of the functions of some modules according to the embodiments of this disclosure. This disclosure can also be implemented as a partial or complete device program (such as a computer program and a computer program product) for executing the method described herein. The program implementing this disclosure can be stored at a computer-readable medium or can have the form of one or more signals. Such signals can be downloaded from internet websites, provided on carrier signals, or in any other form.

It should be noted that the above embodiments illustrate this disclosure rather than limit it, and Ordinary technical personnel in this field can design alternative embodiments without departing from the scope of the attached claims. In the claims, any reference symbol between parentheses should not be constructed as a restriction on the claims. This disclosure can be implemented with the help of hardware consisting of several different components and with the help of appropriately programmed computers. Among the unit claims that list several devices, several of these devices can be specifically embodied through the same hardware item. The use of words first, second, and third does not indicate any order. These words can be interpreted as names.

The above is only the specific implementation method or explanation of the specific implementation method of this disclosure. The protection scope of this disclosure is not limited to this. Any technical personnel familiar with the technical field can easily think of changes or replacements within the technical scope disclosed in this disclosure, and those changes or replacements should fall into the protection scope of this disclosure. The protection scope of this disclosure shall be based on the protection scope of the claims.

## Claims

1. A wearable mobile monitoring device, **characterized in that**, comprising a sensor, a processor, a first wireless communication unit and a second wireless communication unit; wherein:
the sensor is configured to acquire a signal which characterizes at least one vital sign parameter of a human body; and
the processor is configured to perform following operations:
obtaining first monitoring data based on the signal which is acquired by the sensor;
controlling the mobile monitoring device to receive, from a mobile monitoring unit which is worn on the human body, second monitoring data, through first communicative connection, which is established between the first wireless communication unit and the mobile monitoring unit;
transmitting, under a first preset condition to a near-end device which is apart from the human body, the first monitoring data and the second monitoring data, through second communicative connection, which is established between the first wireless communication unit and the near-end device; wherein the second communicative connection and the first communicative connection are based on a same wireless communication technology, the near-end device is located within a preset range; and
transmitting, under a second preset condition to a far-end device, the first monitoring data and the second monitoring data, through third communicative connection, which is established between the second wireless communication unit and the far-end device; wherein the third communicative connection and the first communicative connection are based on different wireless communication technologies, and the far-end device is located outside the preset range.

2. The mobile monitoring device according to claim 1, **characterized in that**, the first preset condition comprises: a communication quality for current communication between the mobile monitoring device and the near-end device through the first wireless communication unit, satisfies a preset standard.

3. The mobile monitoring device according to claim 1, **characterized in that**, the second preset condition comprises: a communication quality for current communication between the mobile monitoring device and the near-end device through the first wireless communication unit, does not satisfy a preset standard.

4. The mobile monitoring device according to any one of claims 1-3, **characterized in that**, when transmitting the first monitoring data and the second monitoring data, the processor is specifically configured to perform following operations:
transmitting, under a third preset condition, the first monitoring data and the second monitoring data, through the second communicative connection or the third communicative connection in a continuous transmission mode; or
transmitting, under a preset condition which is other than the third present condition, the first monitoring data and the second monitoring data, through the second communicative connection or the third communicative connection in a continuous transmission mode or a discontinuous transmission mode;
wherein the continuous transmission mode refers to transmitting data in multiple continuous transmission cycles, and the discontinuous transmission mode refers to transmitting data only in a portion of transmission cycle(s) of multiple continuous transmission cycles.

5. The mobile monitoring device according to claim 4, **characterized in that**, the third preset condition comprises at least one of a condition which reflects a human health risk, a condition which reflects a parameter transmission mode, and a condition which reflects a user instruction.

6. The mobile monitoring device according to claim 5, **characterized in that**, the condition which reflects a human health risk comprises:
a monitoring time of the mobile monitoring device for the human body, does not reach a preset time threshold; and/or
the first monitoring data and/or the second monitoring data indicate(s) that the human body is currently in a risk state.

7. The mobile monitoring device according to claim 5, **characterized in that**, the condition which reflects a user instruction comprises:
a user instruction, which instructs to transmit the first monitoring data and the second monitoring data in the continuous transmission mode, is received by the mobile monitoring device.

8. The mobile monitoring device according to claim 5, **characterized in that**, the condition which reflects a parameter transmission mode comprises:
the first monitoring data and the second monitoring data are transmitted through the first wireless communication unit.

9. The mobile monitoring device according to claim 1, **characterized in that**, the first wireless communication unit comprises a Bluetooth communication module or a Medical Body Area Network communication module.

10. The mobile monitoring device according to claim 1, **characterized in that**, the second wireless communication unit comprises at least one of a WIFI communication module, a WMTS communication module, and a cellular network communication module.

11. The mobile monitoring device according to claim 1, **characterized in that**, further comprising a memory, wherein the processor is further configured to perform a following operation:
storing, in the memory, the first monitoring data and the second monitoring data, when the second communicative connection between the mobile monitoring device and the near-end device is disconnected.

12. The mobile monitoring device according to claim 11, **characterized in that**, the processor is further configured to perform a following operation:
transmitting, to the near-end device, the first monitoring data and the second monitoring data which are stored in the memory, through the second communicative connection between the mobile monitoring device and the near-end device, when the second communicative connection is restored.

13. The mobile monitoring device according to claim 1, **characterized in that**, further comprising a display screen, which is configured to display the first monitoring data and/or the second monitoring data.

14. The mobile monitoring device according to claim 1, **characterized in that**, the near-end device comprises a monitor which is in a same ward as the mobile monitoring device, and the far-end device comprises a central station.

15. The mobile monitoring device according to claim 1, **characterized in that**, further comprising a proximity-based communication unit, wherein the processor is further configured to perform following operations:
acquiring, from the mobile monitoring unit, first connection information, in a contactless manner through the proximity-based communication unit, or transmitting, to the mobile monitoring unit, second connection information, in a contactless manner through the proximity-based communication unit; and establishing, based on the first connection information or the second connection information, the first communicative connection with the mobile monitoring unit, through the first wireless communication unit; and/or
acquiring, from the near-end device, third connection information, in a contactless manner through the proximity-based communication unit, or transmitting, to the near-end device, fourth connection information, in a contactless manner through the proximity-based communication unit; and establishing, based on the third connection information or the fourth connection information, the second communicative connection with the near-end device, through the first wireless communication unit.

16. The mobile monitoring device according to claim 15, **characterized in that**:
establishing, based on the first connection information or the second connection information, the first communicative connection with the mobile monitoring unit, through the first wireless communication unit, comprises:
broadcasting, through the first wireless communication unit, the first connection information or the second connection information, so as to enable the mobile monitoring unit to establish the first communicative connection with the mobile monitoring device, when the first connection information or the second connection information is detected by the mobile monitoring unit; or monitoring information through the first wireless communication unit, and establishing the first communicative connection with the mobile monitoring unit, when the first connection information or the second connection information, which is broadcasted by the mobile monitoring unit, is detected by the first wireless communication unit;
establishing, based on the third connection information or the fourth connection information, the second communicative connection with the near-end device, through the first wireless communication unit, comprises:
broadcasting, through the first wireless communication unit, the third connection information or the fourth connection information, so as to enable the near-end device to establish the second communicative connection with the mobile monitoring device, when the third connection information or the fourth connection information is detected by the near-end device; or monitoring information through the first wireless communication unit, and establishing the second communicative connection with the near-end device, when the third connection information or the fourth connection information, which is broadcasted by the near-end device, is detected by the first wireless communication unit.

17. The mobile monitoring device according to claim 15 or claim 16, **characterized in that**:
the first connection information comprises at least one of: a device identifier or a serial number of the mobile monitoring unit; one of a communication channel identifier, an IP address, an MAC address, a service set identifier, WEP/WPA/WPA2/WPA3 protocol information, EAP information, which are determined by the mobile monitoring unit; and a random pairing code which is generated by the mobile monitoring unit;
the second connection information and the fourth connection information include at least one of a device identifier or a serial number of the mobile monitoring device; one of a communication channel identifier, an IP address, an MAC address, a service set identifier, WEP/WPA/WPA2/WPA3 protocol information, EAP information, which are determined by the mobile monitoring device; and a random pairing code which is generated by the mobile monitoring device;
the third connection information comprises at least one of a device identifier or a serial number of the near-end device; one of a communication channel identifier, an IP address, an MAC address, a service set identifier, WEP/WPA/WPA2/WPA3 protocol information, EAP information, which are determined by the near-end device; and a random pairing code which is generated by the near-end device.

18. A monitoring system, **characterized in that**, comprising: a wearable mobile monitoring device and a mobile monitoring unit which are worn on a human body; and a near-end device and a far-end device which are apart from the human body; wherein:
the mobile monitoring device is configured to monitor first monitoring data of the human body;
the mobile monitoring unit is configured to monitor second monitoring data of the human body;
the near-end device and the far-end device are configured to obtain the first monitoring data and the second monitoring data;
the mobile monitoring device is further configured to perform following operations:
receiving, from the mobile monitoring unit, the second monitoring data, through first communicative connection which is established with the mobile monitoring unit;
transmitting, under a first preset condition to the near-end device, the first monitoring data and the second monitoring data, through second communicative connection, which is established with the near-end device; wherein the second communicative connection and the first communicative connection are based on a same wireless communication technology, the near-end device is located within a preset range; and
transmitting, under a second preset condition to the far-end device, the first monitoring data and the second monitoring data, through third communicative connection, which is established with the far-end device; wherein the third communicative connection and the first communicative connection are based on different wireless communication technologies, and the far-end device is located outside the preset range.

19. The monitoring system according to claim 18, **characterized in that**, the first preset condition comprises: a communication quality for current communication between the mobile monitoring device and the near-end device through the second communicative connection, satisfies a preset standard.

20. The monitoring system according to claim 18, **characterized in that**, the second preset condition comprises: a communication quality for current communication between the mobile monitoring device and the near-end device through the second communicative connection, does not satisfy a preset standard.

21. The monitoring system according to any one of claims 18-20, **characterized in that**, when transmitting the first monitoring data and the second monitoring data, the mobile monitoring device is specifically configured to perform following operations:
transmitting, under a third preset condition, the first monitoring data and the second monitoring data, through the second communicative connection or the third communicative connection in a continuous transmission mode; or
transmitting, under a preset condition which is other than the third present condition, the first monitoring data and the second monitoring data, through the second communicative connection or the third communicative connection in a continuous transmission mode or a discontinuous transmission mode;
wherein the continuous transmission mode refers to transmitting data in multiple continuous transmission cycles, and the discontinuous transmission mode refers to transmitting data only in a portion of transmission cycle(s) of multiple continuous transmission cycles.

22. The monitoring system according to claim 22, **characterized in that**, the third preset condition comprises at least one of a condition which reflects a human health risk, a condition which reflects a parameter transmission mode, and a condition which reflects a user instruction.

23. The monitoring system according to claim 22, **characterized in that**, the condition which reflects a human health risk comprises:
a monitoring time of the mobile monitoring device for the human body, does not reach a preset time threshold; and/or
the first monitoring data and/or the second monitoring data indicate(s) that the human body is currently in a risk state.

24. The monitoring system according to claim 22, **characterized in that**, the condition which reflects a user instruction comprises:
a user instruction, which instructs to transmit the first monitoring data and the second monitoring data in the continuous transmission mode, is received by the mobile monitoring device.

25. The monitoring system according to claim 22, **characterized in that**, the condition which reflects a parameter transmission mode comprises:
the first monitoring data and the second monitoring data are transmitted through a first wireless communication unit of the mobile monitoring device, wherein the first wireless communication unit comprises a Bluetooth communication module or a Medical Body Area Network communication module.

26. The monitoring system according to claim 18, **characterized in that**, the first communicative connection comprises Bluetooth communicative connection or Medical Body Area Network communicative connection.

27. The monitoring system according to claim 18, **characterized in that**, the third communicative connection comprises at least one of WIFI communicative connection, WMTS communicative connection, and cellular network communicative connection.

28. The monitoring system according to claim 18, **characterized in that**, the mobile monitoring device is further configured to perform a following operation:
storing the first monitoring data and the second monitoring data, when the second communicative connection between the mobile monitoring device and the near-end device is disconnected.

29. The monitoring system according to claim 28, **characterized in that**, the mobile monitoring device is further configured to perform a following operation:
transmitting, to the near-end device, the first monitoring data and the second monitoring data which are stored, through the second communicative connection between the mobile monitoring device and the near-end device, when the second communicative connection is restored.

30. The monitoring system according to claim 18, **characterized in that**, the mobile monitoring device is further configured to perform a following operation:
displaying the first monitoring data and/or the second monitoring data.

31. The monitoring system according to claim 18, **characterized in that**, the near-end device comprises a monitor which is in a same ward as the mobile monitoring device; and the far-end device comprises a central station.

32. The monitoring system according to claim 18, **characterized in that**, the mobile monitoring device is further configured to perform following operations:
acquiring, from the mobile monitoring unit, first connection information, in a contactless manner, or transmitting, to the mobile monitoring unit, second connection information, in a contactless manner; and establishing, based on the first connection information or the second connection information, the first communicative connection with the mobile monitoring unit; and/or
acquiring, from the near-end device, third connection information, in a contactless manner, or transmitting, to the near-end device, fourth connection information, in a contactless manner; and establishing, based on the third connection information or the fourth connection information, the second communicative connection with the near-end device.

33. The monitoring system according to claim 32, **characterized in that**:
establishing, based on the first connection information or the second connection information, the first communicative connection with the mobile monitoring unit, comprises:
broadcasting the first connection information or the second connection information, so as to enable the mobile monitoring unit to establish the first communicative connection with the mobile monitoring device, when the first connection information or the second connection information is detected by the mobile monitoring unit; or monitoring information, and establishing the first communicative connection with the mobile monitoring unit, when the first connection information or the second connection information, which is broadcasted by the mobile monitoring unit, is detected;
establishing, based on the third connection information or the fourth connection information, the second communicative connection with the near-end device, comprises:
broadcasting the third connection information or the fourth connection information, so as to enable the near-end device to establish the second communicative connection with the mobile monitoring device, when the third connection information or the fourth connection information is detected by the near-end device; or monitoring information, and establishing the second communicative connection with the near-end device, when the third connection information or the fourth connection information, which is broadcasted by the near-end device, is detected.

34. The monitoring system according to claim 32 or claim 33, **characterized in that**:
the first connection information comprises at least one of: a device identifier or a serial number of the mobile monitoring unit; one of a communication channel identifier, an IP address, an MAC address, a service set identifier, WEP/WPA/WPA2/WPA3 protocol information, EAP information, which are determined by the mobile monitoring unit; and a random pairing code which is generated by the mobile monitoring unit;
the second connection information and the fourth connection information include at least one of a device identifier or a serial number of the mobile monitoring device; one of a communication channel identifier, an IP address, an MAC address, a service set identifier, WEP/WPA/WPA2/WPA3 protocol information, EAP information, which are determined by the mobile monitoring device; and a random pairing code which is generated by the mobile monitoring device;
the third connection information comprises at least one of a device identifier or a serial number of the near-end device; one of a communication channel identifier, an IP address, an MAC address, a service set identifier, WEP/WPA/WPA2/WPA3 protocol information, EAP information, which are determined by the near-end device; and a random pairing code which is generated by the near-end device.

35. A data transmission method which is applicable to a wearable mobile monitoring device, **characterized in that**, comprising:
acquiring a signal which characterizes at least one vital sign parameter of a human body;
obtaining first monitoring data based on the signal;
receiving, from a mobile monitoring unit which is worn on the human body, second monitoring data, through first communicative connection which is established with the mobile monitoring unit;
transmitting, under a first preset condition to a near-end device which is apart from the human body, the first monitoring data and the second monitoring data, through second communicative connection, which is established with the near-end device; wherein the second communicative connection and the first communicative connection are based on a same wireless communication technology, the near-end device is located within a preset range; and
transmitting, under a second preset condition to a far-end device, the first monitoring data and the second monitoring data, through third communicative connection, which is established with the far-end device; wherein the third communicative connection and the first communicative connection are based on different wireless communication technologies, and the far-end device is located outside the preset range.

36. The data transmission method according to claim 35, **characterized in that**, the first preset condition comprises: a communication quality for current communication between the mobile monitoring device and the near-end device through the second communicative connection, satisfies a preset standard.

37. The data transmission method according to claim 35, **characterized in that**, the second preset condition comprises: a communication quality for current communication between the mobile monitoring device and the near-end device through the second communicative connection, does not satisfy a preset standard.

38. The data transmission method according to any one of claims 35-37, **characterized in that**, transmitting the first monitoring data and the second monitoring data, specifically comprises:
transmitting, under a third preset condition, the first monitoring data and the second monitoring data, through the second communicative connection or the third communicative connection in a continuous transmission mode; or
transmitting, under a preset condition which is other than the third present condition, the first monitoring data and the second monitoring data, through the second communicative connection or the third communicative connection in a continuous transmission mode or a discontinuous transmission mode;
wherein the continuous transmission mode refers to transmitting data in multiple continuous transmission cycles, and the discontinuous transmission mode refers to transmitting data only in a portion of transmission cycle(s) of multiple continuous transmission cycles.

39. The data transmission method according to claim 38, **characterized in that**, the third preset condition comprises at least one of: a condition which reflects a human health risk, a condition which reflects a parameter transmission mode, and a condition which reflects a user instruction.

40. The data transmission method according to claim 39, **characterized in that**, the condition which reflects a human health risk comprises:
a monitoring time of the mobile monitoring device for the human body, does not reach a preset time threshold; and/or
the first monitoring data and/or the second monitoring data indicate(s) that the human body is currently in a risk state.

41. The data transmission method according to claim 39, **characterized in that**, the condition which reflects a user instruction comprises:
a user instruction, which instructs to transmit the first monitoring data and the second monitoring data in the continuous transmission mode, is received by the mobile monitoring device.

42. The data transmission method according to claim 39, **characterized in that**, the condition which reflects a parameter transmission mode comprises:
the first monitoring data and the second monitoring data are transmitted through a first wireless communication unit of the mobile monitoring device, wherein the first wireless communication unit comprises a Bluetooth communication module or a Medical Body Area Network communication module.

43. The data transmission method according to claim 35, **characterized in that**, the first communicative connection comprises Bluetooth communicative connection or Medical Body Area Network communicative connection.

44. The data transmission method according to claim 35, **characterized in that**, the third communicative connection comprises at least one of: WIFI communicative connection, WMTS communicative connection, and cellular network communicative connection.

45. The data transmission method according to claim 35, **characterized in that**, further comprising:
storing the first monitoring data and the second monitoring data, when the second communicative connection with the near-end device is disconnected.

46. The data transmission method according to claim 45, **characterized in that**, further comprising:
transmitting, to the near-end device, the first monitoring data and the second monitoring data which are stored, through the second communicative connection with the near-end device, when the second communicative connection is restored.

47. The data transmission method according to claim 35, **characterized in that**, further comprising:
displaying the first monitoring data and/or the second monitoring data.

48. The data transmission method according to claim 35, **characterized in that**, the near-end device comprises a monitor which is in a same ward as the mobile monitoring device; and the far-end device comprises a central station.

49. The data transmission method according to claim 35, **characterized in that**, further comprising:
acquiring, from the mobile monitoring unit, first connection information, in a contactless manner, or transmitting, to the mobile monitoring unit, second connection information, in a contactless manner; and establishing, based on the first connection information or the second connection information, the first communicative connection with the mobile monitoring unit; and/or
acquiring, from the near-end device, third connection information, in a contactless manner, or transmitting, to the near-end device, fourth connection information, in a contactless manner; and establishing, based on the third connection information or the fourth connection information, the second communicative connection with the near-end device.

50. The data transmission method according to claim 49, **characterized in that**:
establishing, based on the first connection information or the second connection information, the first communicative connection with the mobile monitoring unit, comprises:
broadcasting the first connection information or the second connection information, so as to enable the mobile monitoring unit to establish the first communicative connection with the mobile monitoring device, when the first connection information or the second connection information is detected by the mobile monitoring unit; or monitoring information, and establishing the first communicative connection with the mobile monitoring unit, when the first connection information or the second connection information, which is broadcasted by the mobile monitoring unit, is detected;
establishing, based on the third connection information or the fourth connection information, the second communicative connection with the near-end device, comprises:
broadcasting the third connection information or the fourth connection information, so as to enable the near-end device to establish the second communicative connection with the mobile monitoring device, when the third connection information or the fourth connection information is detected by the near-end device; or monitoring information, and establishing the second communicative connection with the near-end device, when the third connection information or the fourth connection information, which is broadcasted by the near-end device, is detected.

51. The data transmission method according to claim 49 or claim 50, **characterized in that**:
the first connection information comprises at least one of: a device identifier or a serial number of the mobile monitoring unit; one of a communication channel identifier, an IP address, an MAC address, a service set identifier, WEP/WPA/WPA2/WPA3 protocol information, EAP information, which are determined by the mobile monitoring unit; and a random pairing code which is generated by the mobile monitoring unit;
the second connection information and the fourth connection information include at least one of a device identifier or a serial number of the mobile monitoring device; one of a communication channel identifier, an IP address, an MAC address, a service set identifier, WEP/WPA/WPA2/WPA3 protocol information, EAP information, which are determined by the mobile monitoring device; and a random pairing code which is generated by the mobile monitoring device;
the third connection information comprises at least one of a device identifier or a serial number of the near-end device; one of a communication channel identifier, an IP address, an MAC address, a service set identifier, WEP/WPA/WPA2/WPA3 protocol information, EAP information, which are determined by the near-end device; and a random pairing code which is generated by the near-end device.
